# EUROPEAN PATENT SPECIFICATION

(11) **EP 2 558 113 B1**
(45) Date of publication and mention of the grant of the patent: **11.01.2017**
(21) Application number: 11769611.2
(22) Date of filing: 14.04.2011
(51) Int. Cl.: A61K 38/17, A61K 38/16, C12N 15/861, C12N 15/12, A61P 11/00

(54) **SERCA2 THERAPEUTIC COMPOSITIONS AND METHODS OF USE**
THERAPEUTISCHE SERCA2-ZUSAMMENSETZUNGEN UND VERFAHREN ZU IHRER VERWENDUNG
COMPOSITIONS THÉRAPEUTIQUES DE SERCA2 ET MÉTHODES D'UTILISATION

(30) Priority: 15.04.2010 US 324670 P
(43) Date of publication of application: 20.02.2013
(73) Proprietor: Celladon Corporation, La Jolla, CA 92037 (US); Mount Sinai School Of Medicine, New York, NY 10028 (US)
(72) Inventor: HAJJAR, Roger, J., Tenafly NJ 07670 (US); KAWASE, Yoshiaki, New York NY 10028 (US); LADAGE, Dennis, New York NY 10029 (US); ZSEBO, Krisztina, La Jolla, CA (US)
(74) Representative: Cornish, Kristina Victoria Joy
(86) International application number: PCT/US2011/032551
(87) International publication number: WO 2011/130552

(56) References cited:
- WO-A1-2010/040130
- US-A1- 2006 018 841
- Zlatko I Pozeg ET AL: "In Vivo Gene Transfer of the O2-Sensitive Potassium Channel Kv1.5 Reduces Pulmonary Hypertension and Restores Hypoxic Pulmonary Vasoconstriction in Chronically Hypoxic Rats", Circulation, 14 April 2003 (2003-04-14), pages 2037-2044, XP055096966, United States DOI: 10.1161/01.CIR.0000062688.76508.B3 Retrieved from the Internet: URL:http://circ.ahajournals.org/cgi/conten t/abstract/107/15/2037 [retrieved on 2014-01-17]
- SAKAI S ET AL: "Pulmonary hypertension caused by congestive heart failure is ameliorated by long-term application of an endothelin receptor antagonist : Increased expression of endothelin -1 messenger ribonucleic acid and endothelin -1-like immunoreactivity in the lung in congestive heart failure in rats", JOURNAL OF THE AMERICAN COLLEGE OF CARDIOLOGY, ELSEVIER, NEW YORK, NY, US, vol. 28, no. 6, 15 November 1996 (1996-11-15), pages 1580-1588, XP002092833, ISSN: 0735-1097, DOI: 10.1016/S0735-1097(96)00336-1
- MEHRAN MANDEGAR ET AL: "Cellular and molecular mechanisms of pulmonary vascular remodeling: role in the development of pulmonary hypertension", MICROVASCULAR RESEARCH, vol. 68, no. 2, 1 September 2004 (2004-09-01), pages 75-103, XP055096795, ISSN: 0026-2862, DOI: 10.1016/j.mvr.2004.06.001
- ANGEL COGOLLUDO ET AL: "Mechanisms Controlling Vascular Tone in Pulmonary Arterial Hypertension: Implications for Vasodilator Therapy", PHARMACOLOGY, vol. 79, no. 2, 1 January 2007 (2007-01-01), pages 65-75, XP055096796, ISSN: 0031-7012, DOI: 10.1159/000097754
- HOSHIJIMA MASAHIKO ET AL: "Chronic suppression of heart-failure progression by a pseudophosphorylated mutant of phospholamban via in vivo cardiac rAAV gene delivery", NATURE MEDICINE, NATURE PUBLISHING GROUP, NEW YORK, NY, US, vol. 8, no. 8, 1 August 2002 (2002-08-01), pages 864-871, XP002303162, ISSN: 1078-8956
- Mehran Mandegar ET AL: "Role of K+ channels in pulmonary hypertension", Vascular pharmacology, 1 January 2002 (2002-01-01), pages 25-33, XP055096649, United States DOI: 10.1016/S1537-1891(02)00123-4 Retrieved from the Internet: URL:http://www.sciencedirect.com/science/a rticle/pii/S1537189102001234
- WARD J P ET AL: "Mechanisms of hypoxic pulmonary vasoconstriction and their roles in pulmonary hypertension: new findings for an old problem", CURRENT OPINION IN PHARMACOLOGY, ELSEVIER SCIENCE PUBLISHERS, NL, vol. 9, no. 3, 1 June 2009 (2009-06-01), pages 287-296, XP026140340, ISSN: 1471-4892, DOI: 10.1016/J.COPH.2009.02.006 [retrieved on 2009-03-16]
- EHLERMANN P ET AL: "Right ventricular upregulation of the Ca<2+> binding protein S100A1 in chronic pulmonary hypertension", BIOCHIMICA ET BIOPHYSICA ACTA. MOLECULAR BASIS OF DISEASE, AMSTERDAM, NL, vol. 1500, no. 2, 21 February 2000 (2000-02-21), pages 249-255, XP004276950, ISSN: 0925-4439, DOI: 10.1016/S0925-4439(99)00106-4
- Muthu Periasamy ET AL: "SERCA pump isoforms: Their role in calcium transport and disease", MUSCLE & NERVE, vol. 35, no. 4, 1 April 2007 (2007-04-01), pages 430-442, XP055229983, US ISSN: 0148-639X, DOI: 10.1002/mus.20745

## Description

### BACKGROUND OF THE INVENTION

### FIELD OF INVENTION

The present invention relates generally to treating pulmonary disease, and more specifically, to treating pulmonary hypertension in a subject by delivering a polynucleotide encoding sarcoplasmic reticulum Ca⁺⁺ ATPase (SERCA2a) protein in an AAV expression vector.

### BACKGROUND INFORMATION

Pulmonary hypertension (PH or PHT) is an increase in blood pressure in the pulmonary artery, pulmonary vein, or pulmonary capillaries, together known as the lung vasculature, leading to shortness of breath, dizziness, fainting, and other symptoms, all of which are exacerbated by exertion. Pulmonary hypertension can be a severe disease with a markedly decreased exercise tolerance and heart failure.

Whatever the initial cause, pulmonary arterial hypertension involves the vasoconstriction or tightening of blood vessels connected to and within the lungs. This makes it harder for the heart to pump blood through the lungs, much as it is harder to make water flow through a narrow pipe as opposed to a wide one. Over time, the affected blood vessels become both stiffer and thicker, in a process known as fibrosis. This further increases the blood pressure within the lungs and impairs their blood flow. In addition, the increased workload of the heart causes thickening and enlargement of the right ventricle, making the heart less able to pump blood through the lungs, causing right heart failure. As the blood flowing through the lungs decreases, the left side of the heart receives less blood. This blood may also carry less oxygen than normal. Therefore it becomes harder and harder for the left side of the heart to pump to supply sufficient oxygen to the rest of the body, especially during physical activity.

Pulmonary venous hypertension does not involve an obstruction to blood flow in the lungs. Instead, the left heart fails to pumps blood efficiently, leading to pooling of blood in the lungs. This causes pulmonary edema and pleural effusions.

In hypoxic pulmonary hypertension, the low levels of oxygen are thought to cause vasoconstriction or tightening of pulmonary arteries. This leads to a similar pathophysiology as pulmonary arterial hypertension.

In chronic thromboembolic pulmonary hypertension, the blood vessels are blocked or narrowed with blood clots. Again, this leads to a similar pathophysiology as pulmonary arterial hypertension.

To date, there is no known cure for pulmonary hypertension. Although intravenous prostacyclin (a current treatment regimen for pulmonary hypertension) therapy prolongs survival in patients with PAH, the use of this treatment option is limited by the short half-life of the drug, requirement for a continuous infusion system, and catheter-related complications. As such, current treatment regimens are intended to control symptoms associated with the disease. When the pulmonary hypertension is brought on my another condition, then treatment is usually directed at that underlying disease. Accordingly, the advent of a new therapy which allows continuous production of therapeutic material has been longing.

### SUMMARY OF THE INVENTION

The present invention is based on the seminal discovery that sarcoplasmic reticulum (SR) calcium ⁺⁺ ATpase (SERCA) overexpression prevents remodeling of smooth muscle cells of blood vessels and increases relaxation and production eNOS, which is a well-known vasodilator. As such, overexpression of SERCA2a on the pulmonary artery is used to treat pulmonary hypertension.

In one aspect, the invention provides an adeno-associated viral (AAV) expression vector for use in treating pulmonary hypertension, comprising a sarcoplasmic reticulum (SR) calcium⁺⁺ ATPase (SERCA) transgene, wherein the transgene is SERCA isoform 2a (SERCA2a) and modulates Ca²⁺ ion transport, and wherein expression of the transgene increases host cell function in a subject. Treatment of pulmonary hypertension includes, but is not limited to, decreasing blood pressure in the pulmonary artery and/or the pulmonary vein. The host cells may be associated with pulmonary function, such as pulmonary artery muscle cells, pulmonary vein muscle cells, pulmonary capillary muscle cells, pulmonary arterioles, alveolae and vascular smooth muscle cells. The AAV vector may be serotype 1 (rAAV1), serotype 2 (AAV2) or serotype 8 (rAAV8). The treatment may decrease blood pressure in the pulmonary artery or the pulmonary vein, may result in lower systolic pressure and ventricular diastolic pressure, may improve pulmonary vascular remodeling, may decrease proliferation of vascular smooth muscle cells, or may increase production of eNOS from endothelial cells.

The subject may be a mammal, such as a human.

The vector may be delivered by a method selected from the group consisting of intranasal spray, inhalation, and aerosol delivery. The vector may be delivered in the form of a microparticle.

In another aspect, the invention provides a recombinant adeno-associated virus (AAV) virion for use in treating pulmonary hypertension, wherein (a) the virion comprises an AAV vector comprising a transgene operably linked to control elements that direct expression of the transgene in a host cell, (b) the expression of the transgene improves lung function, and (c) the transgene is sarcoplasmic reticulum (SR) calcium ⁺⁺ ATPase (SERCA) isoform 2a (SERCA2a).

In yet another aspect, the invention provides a recombinant AAV virion (rAAV) for use in treating pulmonary hypertension, the rAAV comprising a polynucleotide encoding a protein, wherein (a) the polynucleotide is operably linked to a control element capable of directing expression of the protein, (b) the rAAV virion is delivered directly into a pulmonary muscle cell of a mammalian subject, (c) the protein is sarcoplasmic reticulum (SR) calcium ⁺⁺ ATPase isoform 2a (SERCA2a) and (d) the protein is expressed at a therapeutically effective level in the muscle cell.

The subject may be a human.

The recombinant AAV virion may be delivered by a method selected from the group consisting of intranasal spray, inhalation, and aerosol delivery. The recombinant AAV virion may be delivered in the form of a microparticle.

### BRIEF DESCRIPTION OF THE DRAWINGS

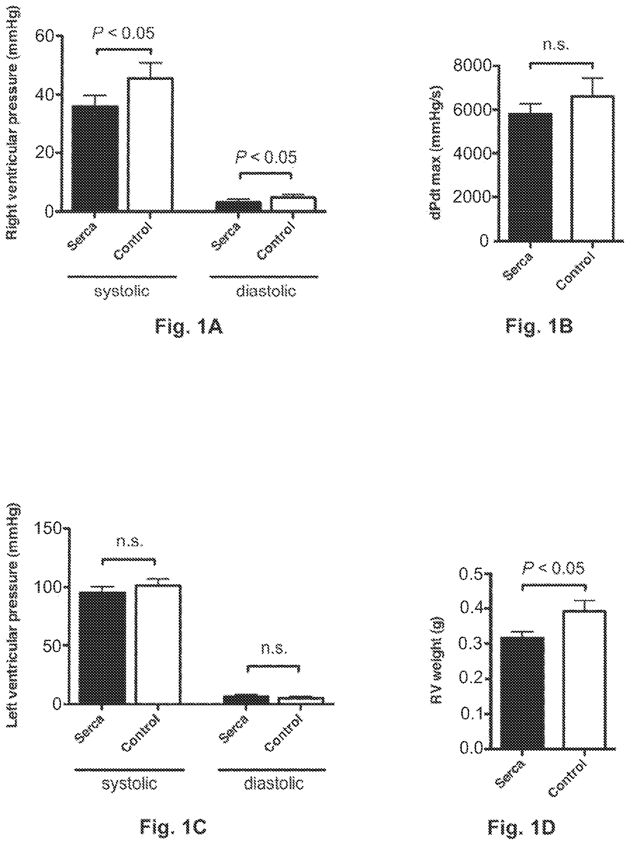

Figures 1A-1D are graphical diagrams showing cardiac pressures and function in rats with pulmonary hypertension after treatment with intra-tracheal AAV1. SERCA2a.

### DETAILED DESCRIPTION OF THE INVENTION

Before the present invention is described, it is to be understood that this invention is not limited to particular compositions, methods, and experimental conditions described, as such compositions, methods, and conditions may vary. It is also to be understood that the terminology used herein is for purposes of describing particular embodiments only, and is not intended to be limiting, since the scope of the present invention will be limited only in the appended claims.

As used in this specification and the appended claims, the singular forms "a", "an", and "the" include plural references unless the context clearly dictates otherwise. Thus, for example, references to "the method" includes one or more methods, and/or steps of the type described herein which will become apparent to those persons skilled in the art upon reading this disclosure and so forth.

Unless defined otherwise, all technical and scientific terms used herein have the same meaning as commonly understood by one of ordinary skill in the art to which this invention belongs. Although any methods and materials similar or equivalent to those described herein can be used in the practice or testing of the invention, the preferred methods and materials are now described.

The practice of the present invention will employ, unless indicated specifically to the contrary, conventional methods of virology, immunology, microbiology, molecular biology and recombinant DNA techniques within the skill of the art, many of which are described below for the purpose of illustration. Such techniques are explained fully in the literature. See, *e*.*g*., Sambrook, et al. Molecular Cloning: A Laboratory Manual (2nd Edition, 1989); Maniatis et al. Molecular Cloning: A Laboratory Manual (1982); DNA Cloning: A Practical Approach, vol. I & II (D. Glover, ed.); Oligonucleotide Synthesis (N. Gait, ed., 1984); Nucleic Acid Hybridization (B. Hames & S. Higgins, eds., 1985); Transcription and Translation (B. Hames & S. Higgins, eds., 1984); Animal Cell Culture (R. Freshney, ed., 1986); Perbal, A Practical Guide to Molecular Cloning (1984).

Pulmonary arterial hypertension (PAH) is a life-threatening disease, with currently no satisfactory established treatments. Gene transfer using adeno-associated virus (AAV) is well known for its safety and ability to express exogenous genes for prolonged periods. SERCA2a overexpression was shown to prevent remodeling of vascular smooth muscle cells (VSMC), decrease proliferation of VSMCs and increase relaxation and production of eNOS from endothelial cells, which is a well-known vasodilator.

Thus, the methods provided herein, utilize a viral vector to deliver the SERCA2 gene or its isoforms directly to the lungs and/or to muscle cells associated with lung function. One aspect of the present invention contemplates transfer of a SERCA2a therapeutic polynucleotide into a cell. Such transfer employs an adeno-associated virus (AAV) vector.

The SERCA2a polynucleotides are incorporated into a viral vector to mediate transfer to a cell. Additional expression constructs encoding other therapeutic agents as described herein may also be transferred via viral transduction using infectious viral particles, for example, by transformation with an adeno-associated virus (AAV) of the present invention. Alternatively, a retrovirus, bovine papilloma virus, an adenovirus vector, a lentiviral vector, a vaccinia virus, a polyoma virus, or an infective virus may be used for such other therapeutic agents. Similarly, nonviral methods which include, but are not limited to, direct delivery of DNA such as by perfusion, naked DNA transfection, liposome mediated transfection, encapsulation, and receptor-mediated endocytosis may be employed for such other therapeutics agents. These techniques are well known to those of skill in the art, and the particulars thereof do not lie at the crux of the present invention and thus need not be exhaustively detailed herein. For example, a viral vector is used for the transduction of pulmonary cells to deliver a therapeutically significant polynucleotide to a cell. The virus may gain access to the interior of the cell by a specific means such as receptor-mediated endocytosis, or by non-specific means such as pinocytosis.

A number of exemplary vectors will now be described. It will be appreciated that the present invention utilizes adeno-associated viral expression vectors or recombinant AAV virions.

### Adeno-associated virus (AAV)

Adeno-associated virus (AAV) has shown promise for delivering genes for gene therapy in clinical trials in humans. As the only viral vector system based on a nonpathogenic and replication-defective virus, recombinant AAV virions have been successfully used to establish efficient and sustained gene transfer of both proliferating and terminally differentiated cells in a variety of tissues.

The AAV genome is a linear, single-stranded DNA molecule containing about 4681 nucleotides. The AAV genome generally comprises an internal nonrepeating genome flanked on each end by inverted terminal repeats (ITRs). The ITRs are approximately 145 base pairs (bp) in length. The ITRs have multiple functions, including as origins of DNA replication, and as packaging signals for the viral genome. The internal nonrepeated portion of the genome includes two large open reading frames, known as the AAV replication (rep) and capsid (cap) genes. The rep and cap genes code for viral proteins that allow the virus to replicate and package into a virion. In particular, a family of at least four viral proteins is expressed from the AAV rep region, Rep 78, Rep 68, Rep 52, and Rep 40, named according to their apparent molecular weight. The AAV cap region encodes at least three proteins, VP1, VP2, and VP3.

AAV has been engineered to deliver genes of interest by deleting the internal nonrepeating portion of the AAV genome (*i.e.,* the rep and cap genes) and inserting a heterologous gene between the ITRs. The heterologous gene is typically functionally or operatively linked to a heterologous promoter (constitutive, cell-specific, or inducible) capable of driving gene expression in the patient's target cells under appropriate conditions. Termination signals, such as polyadenylation sites, can also be included.

As used herein, the term "AAV vector" means a vector derived from an adeno-associated virus serotype, including without limitation, AAV1, AAV2, AAV3, AAV4, AAV5, AAV6, AAV7, AAV8, AAV9, and mutated forms thereof. AAV vectors can have one or more of the AAV wild-type genes deleted in whole or part, preferably the rep and/or cap genes, but retain functional flanking ITR sequences. Despite the high degree of homology, the different serotypes have tropisms for different tissues. The receptor for AAV1 is unknown; however, AAV1 is known to transduce skeletal and smooth muscle more efficiently than AAV2. Without being bound by theory, since most of the studies have been done with pseudotyped vectors in which the vector DNA flanked with AAV2 ITR is packaged into capsids of alternate serotypes, it is clear that the biological differences are related to the capsid rather than to the genomes. Recent evidence indicates that DNA expression cassettes packaged in AAV1 capsids are at least 1 log₁₀ more efficient at transducing cardiomyocytes than those packaged in AAV2 capsids.

Functional ITR sequences are necessary for the rescue, replication and packaging of the AAV virion. Thus, an AAV vector is defined herein to include at least those sequences required in cis for replication and packaging (*e*.*g*., functional ITRs) of the virus. The ITRs need not be the wild-type nucleotide sequences, and may be altered, for example, by the insertion, deletion or substitution of nucleotides, as long as the sequences provide for functional rescue, replication and packaging.

AAV vectors must have one copy of the AAV inverted terminal repeat sequences (ITRs) at each end of the genome in order to be replicated, packaged into AAV particles and integrated efficiently into cell chromosomes. However, the nucleic acid promoted by ITR can be any desired sequence. In one embodiment, the nucleic acid encodes a SERCA2 polypeptide or its isoform (*e*.*g*., SERCA2a), which has a desired function in the cell in which the vector is expressed. For example, the SERCA2 polypeptide increases the control of Ca⁺⁺ storage and regulation in the cell, thereby lowering blood pressure in the arteries of the lungs.

The ITR consists of nucleotides 1 to 145 at the left end of the AAV DNA genome and the corresponding nucleotides 4681 to 4536 (*i*.*e*., the same sequence) at the right hand end of the AAV DNA genome. Thus, AAV vectors must have a total of at least 300 nucleotides of the terminal sequence. So, for packaging large coding regions into AAV vector particles, it is important to develop the smallest possible regulatory sequences, such as transcription promoters and polyA addition signal. In this system, the adeno-associated viral vector comprising the inverted terminal repeat (ITR) sequences of adeno-associated virus and a nucleic acid encoding SERCA (*e*.*g*., SERCA2), its isoforms, fragments and/or variants, wherein the inverted terminal repeat sequences promote expression of the nucleic acid in the absence of another promoter.

Accordingly, as used herein, AAV refers to all serotypes of AAV (*i*.*e*., 1-9) and mutated forms thereof. Thus, it is routine in the art to use the ITR sequences from other serotypes of AAV since the ITRs of all AAV serotypes are expected to have similar structures and functions with regard to replication, integration, excision and transcriptional mechanisms.

AAV is also a helper-dependent virus. That is, it requires coinfection with a helper virus (*e*.*g*., adenovirus, herpesvirus or vaccinia), in order to form AAV virions. In the absence of coinfection with a helper virus, AAV establishes a latent state in which the viral genome inserts into a host cell chromosome, but infectious virions are not produced. Subsequent infection by a helper virus "rescues" the integrated genome, allowing it to replicate and package its genome into an infectious AAV virion. While AAV can infect cells from different species, the helper virus must be of the same species as the host cell. Thus, for example, human AAV will replicate in canine cells coinfected with a canine adenovirus.

The term "AAV helper functions" refer to AAV-derived coding sequences which can be expressed to provide AAV gene products that, in turn, function in trans for productive AAV replication. Thus, AAV helper functions include both of the major AAV open reading frames (ORFs), rep and cap. The Rep expression products have been shown to possess many functions, including, among others: recognition, binding and nicking of the AAV origin of DNA replication; DNA helicase activity; and modulation of transcription from AAV (or other heterologous) promoters. The Cap expression products supply necessary packaging functions. AAV helper functions are used herein to complement AAV functions in trans that are missing from AAV vectors.

Accordingly, the term "AAV helper construct" refers generally to a nucleic acid molecule that includes nucleotide sequences providing AAV functions deleted from an AAV vector which is to be used to produce a transducing vector for delivery of a nucleotide sequence of interest. AAV helper constructs are commonly used to provide transient expression of AAV rep and/or cap genes to complement missing AAV functions that are necessary for lytic AAV replication; however, helper constructs lack AAV ITRs and can neither replicate nor package themselves. AAV helper constructs can be in the form of a plasmid, phage, transposon, cosmid, virus, or virion. A number of AAV helper constructs and vectors that encode Rep and/or Cap expression products have been described.

Typically, recombinant AAV (rAAV) virus is made by cotransfecting a plasmid containing the gene of interest flanked by the two AAV terminal repeats and/or an expression plasmid containing the wild-type AAV coding sequences without the terminal repeats, for example pIM45. The cells are also infected and/or transfected with adenovirus and/or plasmids carrying the adenovirus genes required for AAV helper function. rAAV virus stocks made in such fashion are contaminated with adenovirus which must be physically separated from the rAAV particles (for example, by cesium chloride density centrifugation or column chromatography). Alternatively, adenovirus vectors containing the AAV coding regions and/or cell lines containing the AAV coding regions and/or some or all of the adenovirus helper genes could be used. Cell lines carrying the rAAV DNA as an integrated provirus can also be used.

As used herein, the term "accessory functions" refers to non-AAV derived viral and/or cellular functions upon which AAV is dependent for its replication. Thus, the term captures proteins and RNAs that are required in AAV replication, including those moieties involved in activation of AAV gene transcription, stage specific AAV mRNA splicing, AAV DNA replication, synthesis of Cap expression products and AAV capsid assembly. Viral-based accessory functions can be derived from any of the known helper viruses such as adenovirus, herpesvirus (other than herpes simplex virus type-1) and vaccinia virus.

Accordingly, "accessory function vector" refers generally to a nucleic acid molecule that includes nucleotide sequences providing accessory functions. An accessory function vector can be transfected into a suitable host cell, wherein the vector is then capable of supporting AAV virion production in the host cell. Expressly excluded from the term are infectious viral particles as they exist in nature, such as adenovirus, herpesvirus or vaccinia virus particles. Thus, accessory function vectors can be in the form of a plasmid, phage, transposon or cosmid.

In particular, it has been demonstrated that the full-complement of adenovirus genes is not required for accessory helper functions. In particular, adenovirus mutants incapable of DNA replication and late gene synthesis have been shown to be permissive for AAV replication. Similarly, mutants within the E2B and E3 regions have been shown to support AAV replication, indicating that the E2B and E3 regions are probably not involved in providing accessory functions. However, adenoviruses defective in the E1 region, or having a deleted E4 region, are unable to support AAV replication. Thus, E1A and E4 regions are likely required for AAV replication, either directly or indirectly. Other characterized Ad mutants include: E1B; E2A; E2B; E3; and E4. Although studies of the accessory functions provided by adenoviruses having mutations in the E1B coding region have produced conflicting results, recently it has been reported that E1B55k is required for AAV virion production, while E1B19k is not.

Exemplary accessory function vectors include an adenovirus VA RNA coding region, an adenovirus E4 ORF6 coding region, an adenovirus E2A 72 kD coding region, an adenovirus E1A coding region, and an adenovirus E1B region lacking an intact E1B55k coding region.

By "capable of supporting efficient rAAV virion production" is meant the ability of an accessory function vector or system to provide accessory functions that are sufficient to complement rAAV virion production in a particular host cell at a level substantially equivalent to or greater than that which could be obtained upon infection of the host cell with an adenovirus helper virus. Thus, the ability of an accessory function vector or system to support efficient rAAV virion production can be determined by comparing rAAV virion titers obtained using the accessory vector or system with titers obtained using infection with an infectious adenovirus. More particularly, an accessory function vector or system supports efficient rAAV virion production substantially equivalent to, or greater than, that obtained using an infectious adenovirus when the amount of virions obtained from an equivalent number of host cells is not more than about 200 fold less than the amount obtained using adenovirus infection, more preferably not more than about 100 fold less, and most preferably equal to, or greater than, the amount obtained using adenovirus infection.

Hence, by "AAV virion" is meant a complete virus particle, such as a wild-type (wt) AAV virus particle (comprising a linear, single-stranded AAV nucleic acid genome associated with an AAV capsid protein coat). In this regard, single-stranded AAV nucleic acid molecules of either complementary sense, *e*.*g*., "sense" or "antisense" strands, can be packaged into any one AAV virion and both strands are equally infectious.

Similarly, a "recombinant AAV virion," or "rAAV virion" is defined herein as an infectious, replication-defective virus including an AAV protein shell, encapsidating a heterologous nucleotide sequence of interest which is flanked on both sides by AAV ITRs. A rAAV virion is produced in a suitable host cell which has had an AAV vector, AAV helper functions and accessory functions introduced therein. In this manner, the host cell is rendered capable of encoding AAV polypeptides that are required for packaging the AAV vector (containing a recombinant nucleotide sequence of interest) into infectious recombinant virion particles for subsequent gene delivery.

The AAV system useful in the invention, may also include a sequence encoding a selectable marker. The phrase, "selectable marker" or "selectable gene product" as used herein, refers to the use of a gene which may include but is not limited to: bacterial aminoglycoside 3' phosphotransferase gene (also referred to as the neo gene) which confers resistance to the drug G418 in mammalian cells; bacterial hygromycin G phosphotransferase (hyg) gene which confers resistance to the antibiotic hygromycin; and the bacterial xanthine-guanine phosphoribosyl transferase gene (also referred to as the gpt gene) which confers the ability to grow in the presence of mycophenolic acid. In addition, the AAV system of the invention may also include sequences encoding a visual detectable marker, *e*.*g*., green fluorescent protein (GFP) or any other detectable marker standard in the art and can be identified and utilized by one skilled in the art without undue experimentation.

Those of skill in the art have circumvented some of the limitations of adenovirus-based vectors by using adenovirus "hybrid" viruses, which incorporate desirable features from adenovirus as well as from other types of viruses as a means of generating unique vectors with highly specialized properties. For example, viral vector chimeras were generated between adenovirus and adeno-associated virus (AAV). These aspects of the invention do not deviate from the scope of the invention described herein.

Another method for delivery of the polynucleotide for gene therapy involves the use of an adenovirus expression vector. As used herein, the term "adenovirus expression vector" is meant to include those constructs containing adenovirus sequences sufficient (a) to support packaging of the construct and/or (b) to ultimately express a tissue and/or cell-specific construct that has been cloned therein.

The expression vector may comprise a genetically engineered form of adenovirus. Knowledge of the genetic organization of adenovirus, a 36 kb, linear, double-stranded DNA virus, allows substitution of large pieces of adenoviral DNA with foreign sequences up to 7 kb (Grunhaus and Horwitz, Seminar in Virology, 1992; 3:237-252). In contrast to retrovirus, the adenoviral infection of host cells does not result in chromosomal integration because adenoviral DNA can replicate in an episomal manner without potential genotoxicity. Also, adenoviruses are structurally stable, and no genome rearrangement has been detected after extensive amplification.

Adenovirus growth and manipulation is known to those of skill in the art, and exhibits broad host range *in vitro* and *in vivo.* This group of viruses can be obtained in high titers, *e.g.,* 109 to 1011 plaque-forming units per mL, and they are highly infective. The life cycle of adenovirus does not require integration into the host cell genome. The foreign genes delivered by adenovirus vectors are episomal and, therefore, have low genotoxicity to host cells. No side effects have been reported in studies of vaccination with wild-type adenovirus, demonstrating their safety and/or therapeutic potential as *in vivo* gene transfer vectors.

Adenovirus vectors have been used in eukaryotic gene expression and vaccine development. Recently, animal studies suggested that recombinant adenovirus could be used for gene therapy (see, *e.g.,* Stratford-Perricaudet et al., Hum. Gene. Ther., 1991;1:242-256; Rich *et al.,* 1993). Studies in administering recombinant adenovirus to different tissues include muscle injection, peripheral intravenous injections and stereotactic inoculation into the brain. Recombinant adenovirus and adeno-associated virus can both infect and transduce non-dividing human primary cells.

While the use of adenovirus vectors is contemplated, such use in gene therapy trials is currently limited by short-lived transgene expression. (Vassalli G, et al., Int. J. Cardiol., 2003; 90(2-3):229-38). This is due to cellular immunity against adenoviral antigens. Improved "gutless" adenoviral vectors have reduced immunogenicity, yet still are ineffective if maximal expression of the transgene for more than six months is needed or desired for therapeutic effect (Gilbert R, et al., Hum. Mol. Genet., 2003; 12(11):1287-99). AAV vectors have demonstrated long term expression (> 1 year) and are the preferred vector for therapeutic effects where expression is needed long-term (Daly TM, et al., Gene Ther., 2001; 8(17):1291-8).

### Retroviral Vectors

Retroviruses may be chosen as gene delivery vectors due to their ability to integrate their genes into the host genome, transferring a large amount of foreign genetic material, infecting a broad spectrum of species and cell types and for being packaged in special cell-lines.

The retroviral genome contains three genes, gag, pol, and env that code for capsid proteins, polymerase enzyme, and envelope components, respectively. A sequence found upstream from the gag gene contains a signal for packaging of the genome into virions. Two long terminal repeat (LTR) sequences are present at the 5' and 3' ends of the viral genome. These contain strong promoter and enhancer sequences and are also required for integration in the host cell genome.

In order to construct a retroviral vector, a nucleic acid encoding a gene of interest is inserted into the viral genome in the place of certain viral sequences to produce a virus that is replication-defective. In order to produce virions, a packaging cell line is constructed containing the gag, pol, and/or env genes but without the LTR and/or packaging components. When a recombinant plasmid containing a cDNA, together with the retroviral LTR and packaging sequences is introduced into this cell line (by calcium phosphate precipitation for example), the packaging sequence allows the RNA transcript of the recombinant plasmid to be packaged into viral particles, which are then secreted into the culture media. The media containing the recombinant retroviruses is then collected, optionally concentrated, and used for gene transfer. Retroviral vectors are able to infect a broad variety of cell types. However, integration and stable expression require the division of host cells.

### Herpes virus

Because herpes simplex virus (HSV) is neurotropic, it has generated considerable interest in treating nervous system disorders. Moreover, the ability of HSV to establish latent infections in non-dividing neuronal cells without integrating into the host cell chromosome or otherwise altering the host cell's metabolism, along with the existence of a promoter that is active during latency makes HSV an attractive vector. And though much attention has focused on the neurotropic applications of HSV, this vector also can be exploited for other tissues given its wide host range.

Another factor that makes HSV an attractive vector is the size and organization of the genome. Because HSV is large, incorporation of multiple genes or expression cassettes is less problematic than in other smaller viral systems. In addition, the availability of different viral control sequences with varying performance (temporal, strength, etc.) makes it possible to control expression to a greater extent than in other systems. It also is an advantage that the virus has relatively few spliced messages, further easing genetic manipulations.

HSV also is relatively easy to manipulate and can be grown to high titers. Thus, delivery is less of a problem, both in terms of volumes needed to attain sufficient multiplicity of infection (MOI) and in a lessened need for repeat dosing. For a review of HSV as a gene therapy vector, see Glorioso et al., Annu. Rev. Microbiol., 1995; 49:675-710. Avirulent variants of HSV have been developed and are readily available for use in gene therapy contexts (U.S. Pat. No. 5,672,344).

### Lentiviral Vectors

Lentiviruses are complex retroviruses, which, in addition to the common retroviral genes gag, pol, and env, contain other genes with regulatory or structural function. The higher complexity enables the virus to modulate its life cycle, as in the course of latent infection. Some examples of lentivirus include the Human Immunodeficiency Viruses (HIV 1, HIV 2) and the Simian Immunodeficiency Virus (SIV). Lentiviral vectors have been generated by multiply attenuating the HIV virulence genes, for example, the genes env, vif, vpr, vpu and nef are deleted making the vector biologically safe.

Lentiviral vectors are known in the art, see, *e*.*g*., U.S. Pat. Nos. 6,013,516 and 5,994,136. In general, the vectors are plasmid-based or virus-based, and are configured to carry the essential sequences for incorporating foreign nucleic acid, for selection and for transfer of the nucleic acid into a host cell. The gag, pol and env genes of the vectors of interest also are known in the art. Thus, the relevant genes are cloned into the selected vector and then used to transform the target cell of interest.

Recombinant lentivirus capable of infecting a non-dividing cell wherein a suitable host cell is transfected with two or more vectors carrying the packaging functions, namely gag, pol and env, as well as rev and tat is described in U.S. Pat. No. 5,994,136. This describes a first vector that can provide a nucleic acid encoding a viral gag and a pol gene and another vector that can provide a nucleic acid encoding a viral env to produce a packaging cell. Introducing a vector providing a heterologous gene into that packaging cell yields a producer cell which releases infectious viral particles carrying the foreign gene of interest. The env preferably is an amphotropic envelope protein which allows transduction of cells of human and other species.

### Vaccinia Virus Vectors

Vaccinia virus vectors have been used extensively because of the ease of their construction, relatively high levels of expression obtained, wide host range and large capacity for carrying DNA. Vaccinia contains a linear, double-stranded DNA genome of about 186 kb that exhibits a marked "A-T" preference. Inverted terminal repeats of about 10.5 kb flank the genome. The majority of essential genes appear to map within the central region, which is most highly conserved among poxviruses. Estimated open reading frames in vaccinia virus number from 150 to 200. Although both strands are coding, extensive overlap of reading frames is not common.

At least 25 kb can be inserted into the vaccinia virus genome. Prototypical vaccinia vectors contain transgenes inserted into the viral thymidine kinase gene via homologous recombination. Vectors are selected on the basis of a tk-phenotype. Inclusion of the untranslated leader sequence of encephalomyocarditis virus results in a level of expression that is higher than that of conventional vectors, with the transgenes accumulating at 10% or more of the infected cell's protein in 24 h.

### Polyoma Viruses Vectors

The empty capsids of papovaviruses, such as the mouse polyoma virus, have received attention as possible vectors for gene transfer. The use of empty polyoma was first described when polyoma DNA and purified empty capsids were incubated in a cell-free system. The DNA of the new particle was protected from the action of pancreatic DNase. The reconstituted particles were used for transferring a transforming polyoma DNA fragment to rat FIII cells. The empty capsids and reconstituted particles consist of all three of the polyoma capsid antigens VP1, VP2 and VP3. U.S. Pat. No. 6,046,173, discloses the use of a pseudocapsid formed from papovavirus major capsid antigen and excluding minor capsid antigens, which incorporates exogenous material for gene transfer.

### Other Viral Vectors

Other viral vectors may be employed as expression constructs such as vectors derived from viruses such as sindbis virus or cytomegalovirus. They offer several attractive features for various mammalian cells (see, *e*.*g*., Friedmann, Science, 1989; 244:1275-1281; Horwich et al., J. Virol., 1990; 64:642-650).

With the recognition of defective hepatitis B viruses, new insight was gained into the structure-function relationship of different viral sequences. *In vitro* studies showed that the virus could retain the ability for helper-dependent packaging and reverse transcription despite the deletion of up to 80% of its genome (Horwich et al., J. Virol. 64:642-650 (1990)). This suggested that large portions of the genome could be replaced with foreign genetic material. Chang *et al.* introduced the chloramphenicol acetyltransferase (CAT) gene into duck hepatitis R virus genome in the place of the polymerase, surface, and/or pre-surface coding sequences. It was cotransfected with wild-type virus into an avian hepatoma cell line. Culture media containing high titers of the recombinant virus were used to infect primary duckling hepatocytes. Stable CAT gene expression was detected for at least 24 days after transfection (Chang et al., Hepatology 14:134A (1991)).

### Modified Viruses

Nucleic acids to be delivered may be housed within an infective virus that has been engineered to express a specific binding ligand. The virus particle will thus bind specifically to the cognate receptors of the target cell and deliver the contents to the cell. A novel approach designed to allow specific targeting of retrovirus vectors was developed based on the chemical modification of a retrovirus by the chemical addition of lactose residues to the viral envelope. This modification can permit the specific infection of hepatocytes via sialoglycoprotein receptors.

Another approach to targeting of recombinant retroviruses was designed in which biotinylated antibodies against a retroviral envelope protein or against a specific cell receptor were used. The antibodies were coupled via the biotin components by using streptavidin. Using antibodies against major histocompatibility complex class I and/or class II antigens, they demonstrated the infection of a variety of human cells that bore those surface antigens with an ecotropic virus *in vitro.*

### Non-viral Transfer

Several non-viral methods for the transfer of expression constructs into cultured mammalian cells are disclosed herein. Suitable methods for nucleic acid delivery include methods as described herein or as would be known to one of ordinary skill in the art. Such methods include, but are not limited to, direct delivery of "naked" DNA plasmid via the vasculature (U.S. Pat. No. 6,867,196); by liposome mediated transfection (Nicolau and Sene, 1982; Fraley *et al.,* 1979; Nicolau *et al.,* 1987; Wong *et al.,* 1980; Kaneda *et al.,* 1989; Kato *et al.,* 1991) and receptor-mediated transfection (Wu and Wu, 1987; Wu and Wu, 1988); by agitation with silicon carbide fibers (Kaeppler *et al.,* 1990; U.S. Pat. Nos. 5,302,523 and 5,464,765); use of cationic lipids; naked DNA; or by microencapsulated DNA (U.S. Pat. Appl. No. 2005/0037085). Through the application of techniques such as these, target cells or tissue can be stably or transiently transformed.

Once the construct has been delivered into the cell, the nucleic acid encoding the therapeutic gene may be positioned and expressed at different sites. The nucleic acid encoding the therapeutic gene may be stably integrated into the genome of the cell. This integration may be in the cognate location and orientation via homologous recombination (gene replacement) or it may be integrated in a random, non-specific location (gene augmentation). The nucleic acid may be stably maintained in the cell as a separate, episomal segment of DNA. Such nucleic acid segments or "episomes" encode sequences sufficient to permit maintenance and replication independent of or in synchronization with the host cell cycle. How the expression construct is delivered to a cell and where in the cell the nucleic acid remains is dependent on the type of expression construct employed.

The expression construct may be entrapped in a liposome. Liposomes are vesicular structures characterized by a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers. The addition of DNA to cationic liposomes causes a topological transition from liposomes to optically birefringent liquid-crystalline condensed globules. These DNA-lipid complexes are potential non-viral vectors for use in gene therapy.

Liposome-mediated nucleic acid delivery and expression of foreign DNA *in vitro* has been very successful. Using the β-lactamase gene, investigators demonstrated the feasibility of liposome-mediated delivery and expression of foreign DNA in cultured chick embryo, HeLa, and hepatoma cells. Successful liposome-mediated gene transfer in rats after intravenous injection has also been accomplished. Also included are various commercial approaches involving "lipofection" technology.

The liposome may be complexed with a hemagglutinating virus (HVJ). This has been shown to facilitate fusion with the cell membrane and promote cell entry of liposome-encapsulated DNA. The liposome may be complexed or employed in conjunction with nuclear non-histone chromosomal proteins (HMG-1). The liposome may be complexed or employed in conjunction with both HVJ and HMG-I. In that such expression constructs have been successfully employed in transfer and expression of nucleic acid *in vitro* and *in vivo,* then they are applicable for the present invention.

Other vector delivery systems which can be employed to deliver a nucleic acid encoding a therapeutic gene into cells are receptor-mediated delivery vehicles. These take advantage of the selective uptake of macromolecules by receptor-mediated endocytosis in almost all eukaryotic cells. Because of the cell type-specific distribution of various receptors, the delivery can be highly specific (Wu and Wu, 1993). Where liposomes are employed, other proteins which bind to a cell surface membrane protein associated with endocytosis may be used for targeting and/or to facilitate uptake, *e*.*g*., capsid proteins or fragments thereof tropic for a particular cell type, antibodies for proteins which undergo internalization in cycling, and proteins that target intracellular localization and enhance intracellular half-life.

Receptor-mediated gene targeting vehicles generally consist of two components: a cell receptor-specific ligand and a DNA-binding agent. Several ligands have been used for receptor-mediated gene transfer. The most extensively characterized ligands are asialoorosomucoid (ASOR) and transferring (Wagner et al., Proc. Natl. Acad. Sci. 87(9):3410-14 (1990)). A synthetic neoglycoprotein, which recognizes the same receptor as ASOR, has been used as a gene delivery vehicle. Epidermal growth factor (EGF) has also been used to deliver genes to squamous carcinoma cells.

In other embodiments, the delivery vehicle may comprise a ligand and a liposome. For example, investigators have employed lactosyl-ceramide, a galactose-terminal asialganglioside, incorporated into liposomes and observed an increase in the uptake of the insulin gene by hepatocytes. Thus, it is feasible that a nucleic acid encoding a therapeutic gene also may be specifically delivered into a cell type such as cardiac cells, by any number of receptor-ligand systems with or without liposomes.

The expression construct may simply consist of naked recombinant DNA or plasmids. Transfer of the construct may be performed by any of the methods mentioned above which physically or chemically permeabilize the cell membrane. This is applicable particularly for transfer *in vitro,* however, it may be applied for *in vivo* use as well. It is envisioned that therapeutic DNA may also be transferred in a similar manner *in vivo.* Wolff et al. (U.S. Pat. No. 6,867,196) teach that efficient gene transfer into heart tissue can be obtained by injection of plasmid DNA solutions in a vein or artery of the heart. Wolff also teaches the administration of RNA, non-plasmid DNA, and viral vectors.

The vectors useful in the present invention have varying transduction efficiencies. As a result, the AAV expression vector transduces more than, equal to, or at least about 10%, 20%, 30%, 40%, 50%, 55%, 60%, 65%, 70%, 75%, 80%, 85%, 90%, 95%, 99%, 100% of the cells of the targeted vascular territory. More than one AAV expression vector can be used simultaneously, or in sequence. This can be used to transfer more than one polynucleotide, and/or target more than one type of cell. Where multiple vectors or multiple agents are used, more than one transduction/transfection efficiency can result.

Various nucleic acid sequences are embodied in the present invention. As used herein, "nucleic acid" sequence or equivalents thereof refer to a DNA or RNA sequence. The term captures sequences that include any of the known base analogues of DNA and RNA such as, but not limited to 4-acetylcytosine, 8-hydroxy-N6-methyladenosine, aziridinylcytosine, pseudoisocytosine, 5-(carboxyhydroxylmethyl) uracil, 5-fluorouracil, 5-bromouracil, 5-carboxymethylaminomethyl-2-thiouracil, 5-carboxymethyl-aminomethyluracil, dihydrouracil, inosine, N6-isopentenyladenine, 1-methyladenine, 1-methylpseudouracil, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyluracil, 5-methoxyamino-methyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, -uracil-5-oxyacetic acid methylester, uracil-5-oxyacetic acid, pseudouracil, queosine, 2-thiocytosine, and 2,6-diaminopurine.

Other nucleic acid sequences include "control sequences", which refers collectively to promoter sequences, polyadenylation signals, transcription termination sequences, upstream regulatory domains, origins of replication, internal ribosome entry sites ("IRES"), enhancers, and the like, which collectively provide for the replication, transcription and translation of a coding sequence in a recipient cell. Not all of these control sequences need always be present so long as the selected coding sequence is capable of being replicated, transcribed and translated in an appropriate host cell.

Another nucleic acid sequence, is a "promoter" sequence, which is used herein in its ordinary sense to refer to a nucleotide region comprising a DNA regulatory sequence, wherein the regulatory sequence is derived from a gene which is capable of binding RNA polymerase and initiating transcription of a downstream (3'-direction) coding sequence. Transcription promoters can include "inducible promoters" (where expression of a polynucleotide sequence operably linked to the promoter is induced by an analyte, cofactor, regulatory protein, etc.), "repressible promoters" (where expression of a polynucleotide sequence operably linked to the promoter is induced by an analyte, cofactor, regulatory protein, etc.), and "constitutive promoters."

The nucleic acids embodied in the present invention may be "operably linked" to each other or linked to a protein or peptide. As used herein, "operatively linked" refers to an arrangement of elements wherein the components so described are configured so as to perform their usual function. Thus, control sequences operably linked to a coding sequence are capable of effecting the expression of the coding sequence. The control sequences need not be contiguous with the coding sequence, so long as they function to direct the expression thereof. Thus, for example, intervening untranslated yet transcribed sequences can be present between a promoter sequence and the coding sequence and the promoter sequence can still be considered "operably linked" to the coding sequence.

"Synergistic" or have "synergy" refers to an activity of administering combinations of proteins, lipids, nucleic acids, carbohydrates, or chemical compounds that is greater than the additive activity of the proteins, lipids, nucleic acids, carbohydrates, or chemical compounds if administered individually.

The embodiments of the present invention can be "co-administered", which refers to two or more proteins, lipids, nucleic acids, carbohydrates, or chemical compounds of a combination that are administered so that the therapeutic or prophylactic effects of the combination is greater than the therapeutic effect of either proteins, lipids, nucleic acids, carbohydrates, or chemical compounds administered alone. The two or more proteins, lipids, nucleic acids, carbohydrates, or chemical compounds can be administered simultaneously or sequentially. Simultaneously co-administered proteins, lipids, nucleic acids, carbohydrates, or chemical compounds may be provided in one or more pharmaceutically acceptable compositions. Sequential co-administration includes, but is not limited to, instances in which the proteins, lipids, nucleic acids, carbohydrates, or chemical compounds are administered so that each protein, lipid, nucleic acid, carbohydrate, or chemical compound can be present at the treatment site at the same time.

Another aspect of the present invention administers an "adjuvant" or equivalents thereof, which refers to a compound or mixture that enhances the immune response to an antigen. An adjuvant can serve as a tissue depot that slowly releases the antigen and also as a lymphoid system activator that non-specifically enhances the immune response. Often, a primary challenge with an antigen alone, in the absence of an adjuvant, will fail to elicit a humoral or cellular immune response. Adjuvants include, but are not limited to, complete Freund's adjuvant, incomplete Freund's adjuvant, saponin, mineral gels such as aluminum hydroxide, surface active substances such as lysolecithin, pluronic polyols, polyanions, peptides, oil or hydrocarbon emulsions, keyhole limpet hemocyanins, dinitrophenol, and potentially useful human adjuvants such as BCG (bacille Calmette-Guerin) and Corynebacterium parvum. Preferably, the adjuvant is pharmaceutically acceptable.

In a related aspect, the term "molecular adjuvant" is defined as a protein, lipid, nucleic acid, carbohydrate, or chemical compound for which dendritic cells (DCs), macrophages, B cells, T cells, and/or NK cells have a known receptor whose occupancy leads to a defined sequence of intracellular signal transduction and a change in the phenotype resulting in an improvement in the quantity or quality of the ensuing immune response. In a related aspect, the cells as described above are collectively referred to as "immune cells."

Polynucleotides described herein encode for a "polypeptide", for example, SERCA2 or isomers thereof. "Polypeptide" is used in its conventional meaning, *i.e.,* as a sequence of amino acids. The polypeptides are not limited to a specific length of the product; thus, peptides, oligopeptides, and proteins are included within the definition of polypeptide, and such terms may be used interchangeably herein unless specifically indicated otherwise. This term also does not refer to or exclude post-expression modifications of the polypeptide, for example, glycosylations, acetylations, phosphorylations and the like, as well as other modifications known in the art, both naturally occurring and non-naturally occurring. A polypeptide may be an entire protein, or a subsequence thereof. Particular polypeptides of interest in the context of this invention are amino acid subsequences comprising epitopes, *i. e.,* antigenic determinants substantially responsible for the immunogenic properties of a polypeptide and being capable of evoking an immune response.

Accordingly, the present invention utilises polynucleotides encoding a polypeptide called Sarco/Endoplasmic Reticulum Ca²⁺-ATPase isoform 2a (SERCA2a). SERCA resides in the sarcoplasmic reticulum (SR) within muscle cells. Vasoconstriction (*e*.*g*., vascular smooth muscle cells or VSMC) is reported to be dependent on mobilization Ca²⁺ from intracellular stores or the SR. SERCA is a Ca2⁺ ATPase which transfers Ca2⁺ from the cytosol of the cell to the lumen of the sarcoplasmic reticulum (SR) at the expense of ATP hydrolysis. SERCA proteins are encoded by three genes (SERCA1, 2 and 3) located on separate chromosomes. SERCA transcripts are expressed and alternatively spliced in a tissue dependent manner. The resulting mRNA species encode different SERCA protein isoforms and differ in 3'-untranslated regions (UTR). SERCA protein isoforms differ in their Ca²⁺ affinity, resistance to oxidative stress and modulation by sarcolipin, phospholamban (PLB/PLN), and Ca²⁺/calmodulin kinase II.

In one embodiment, polypeptides are defined by structural domains. For example, the signaling domain, which is associated with transduction upon receptor binding and is found in the cytoplasmic compartment of cells, is defined as a region of a protein molecule delimited on the basis of function and is related to a receptor's cytoplasmic substrate.

In another aspect, the present invention uses variants of the polypeptide compositions described herein. Polypeptide variants generally encompassed by the present invention will typically exhibit at least about 70%, 75%, 80%, 85%, 90%, 91%, 92%, 93%, 94%, 95%, 96%, 97%, 98%, or 99% or more identity, along its length, to a polypeptide sequence set forth herein.

SERCA2 polypeptides, include but are not limited to, human GenBank sequences such as AAB29701 (Wuytack et al., J Biol Chem (1994) 269(2):1410-1416); NP_733765 (Lytton and MacLennan, J Biol Chem (1988) 263(29):15023-15031); NP_001672 (Otsu et al., Genomics (1993) 17(2):507-509); AAH35588 (Strausberg et al., Proc Natl Acad Sci USA (2002) 99(26):16899-16903); and mouse GenBank sequences including AAD01889 (Ver Heyen et al., Mamm Genome (2000) 11(2):159-163); NP_033852 (Hsu et al., Biochem Biophys Res Comm (1993) 197(3):1483-1491); CAB41018 (Ver Heyen et al., Mamm Genome (2000) 11(2):159-163); CAB41017 (Id.); CAB72436 (Id.); CAA11450 (Id.); AAH54531 (Strasberg et al., Proc Natl Acad Sci USA (2002) 99(26):16899-16903); AAH54748 (Id.); and other species including NP_957259 (Ebert et al., Proc Natl Acad Sci USA (2005) 102(49):17705-17710); ABG90496 (Wu et al., Silurus lanzhouensis SERCA2, direct submission (23-JUN-2006), Department of Applied Chemistry, College of Science, China Agricultural University, No. 2, Yuanmigyuan West Road, Haidian, Beijing, 100094, China); NP_001025448 (Sehra H., Danio rerio SERCA2, direct submission (7-AUG-2005), Welcome Trust Sanger Institute, Hixton, Cambridgeshire, CB10 1SA, UK); NP_001009216 (Gambel et al., Biochem Biophys Acta (1992) 1131(2):203-206); NP_999030 (Eggermont et al., Biochem J (1989) 260(3):757-761); NP_058986 (Gunteski-Hamblin et al., J Biol Chem (1988) 263(29):15032-15040); CAA37784 (Eggermont et al., Biochem J (1989) 260(3):757-761); and CAA37783 (Id.). All of the above sequences are publicly available.

Within other illustrative embodiments, a SERCA2 polypeptide may be a fusion polypeptide that comprises multiple SERCA2 polypeptides as described herein, or that comprises at least one SERCA2 polypeptide as described herein and an unrelated sequence, such as a known viral protein. A fusion partner may, for example, assist in providing epitopes (an immunological fusion partner), preferably epitopes recognized by humans, or may assist in expressing the protein (an expression enhancer) at higher yields than the native recombinant protein. Certain fusion partners enhance formation of multimers.

Fusion polypeptides may generally be prepared using standard techniques, including chemical conjugation. Preferably, a fusion SERCA2 polypeptide is expressed as a recombinant polypeptide, allowing the production of increased SERCA2 levels, relative to a non-fused polypeptide, in an expression system. Briefly, DNA sequences encoding the polypeptide components may be assembled separately, and ligated into an appropriate expression vector. The 3' end of the DNA sequence encoding one polypeptide component is ligated, with or without a peptide linker, to the 5' end of a DNA sequence encoding the second polypeptide component so that the reading frames of the sequences are in phase. This permits translation into a single fusion polypeptide that retains the biological activity of both component polypeptides.

The present invention also encompasses "peptide" or "peptide portion" or "fragment" or "peptide fragment" and equivalents thereof, which is used broadly herein to mean two or more amino acids linked by a peptide bond. The term "proteolytic fragment" also is used herein to refer to a product that can be produced by a proteolytic reaction on a polypeptide, *i.e.,* a peptide produced upon cleavage of a peptide bond in the polypeptide. Although the term "proteolytic fragment" is used generally herein to refer to a peptide that can be produced by a proteolytic reaction, it should be recognized that the fragment need not necessarily be produced by a proteolytic reaction, but also can be produced using methods of chemical synthesis or methods of recombinant DNA technology, as discussed in greater detail below, to produce a synthetic peptide that is equivalent to a proteolytic fragment.

Further, the term "functional fragment" or "functional portion" or equivalents thereof means that the SERCA2 fragment or peptide has functional SERCA2 activity, for example, a functional fragment or functional proteolytic fragment of SERCA2 or SERCA2a has functional SERCA2 or SERCA2a activity.

Generally, a peptide contains at least about six amino acids, usually contains about ten amino acids, and can contain fifteen or more amino acids, particularly twenty or more amino acids. It should be recognized that the term "peptide" is not used herein to suggest a particular size or number of amino acids comprising the molecule, and that a peptide of the invention can contain up to several amino acid residues or more.

Also, as used herein, the "translation product" or "polypeptide" refers to peptides, polypeptides, oligopeptides and proteins or protein fragments which have a desired biological effect *in vivo* or *in vitro.* As used herein, the term "fragment" refers to a molecule or any peptide subset of the molecule; whereas a "variant" of such molecule refers to a naturally occurring molecule (*e*.*g*., isoform such as SERCA2a) substantially similar to either the entire molecule, or a fragment thereof. In contrast, an "analog" of a molecule refers to a non-natural molecule substantially similar to either the entire molecule or a fragment thereof. All these are contemplated herein so long as *in vivo* delivery of the therapeutic composition containing SERCA2 polypeptide, fragment, or variant to a subject suffering from pulmonary hypertension ameliorates or effectively treats the subject in need thereof.

A peptide linker sequence may be employed to separate the first and second polypeptide components by a distance sufficient to ensure that each polypeptide folds into its secondary and tertiary structures. Such a peptide linker sequence is incorporated into the fusion polypeptide using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn, and Ser residues. Other near neutral amino acids, such as Thr and Ala may also be used in the linker sequence. Amino acid sequences which may be usefully employed as linkers are generally known in the art. The linker sequence may generally be from 1 to about 50 amino acids in length. Linker sequences are not required when the first and second polypeptides have non-essential N-terminal amino acid regions that can be used to separate the functional domains and prevent steric interference.

The ligated DNA sequences are operably linked to suitable transcriptional or translational regulatory elements. The regulatory elements responsible for expression of DNA are located only 5' to the DNA sequence encoding the first polypeptides. Similarly, stop codons required to end translation and transcription termination signals are only present 3' to the DNA sequence encoding the secondary, tertiary, or quaternary, etc., polypeptide *(i.e.,* a stop codon will be present on the ultimate polypeptide depending on the number of distinct polypeptides making up a chimeric protein molecule).

In addition to SERCA2 polypeptides, the present disclosure provides SERCA2 and SERCA2 containing polynucleotide compositions. The terms "DNA" and "polynucleotide" are used interchangeably herein to refer to a DNA molecule that has been isolated free of total genomic DNA of a particular species. "Isolated," as used herein, means that a polynucleotide is substantially away from other coding sequences, and that the DNA molecule does not contain large portions of unrelated coding DNA, such as large chromosomal fragments or other functional genes or polypeptide coding regions. Of course, this refers to the DNA molecule as originally isolated, and does not exclude genes or coding regions later added to the segment by the hand of man.

The phrase "SERCA2 gene" or "SERCA2 transgene" or other SERCA isomers thereof, refers to DNA or RNA and can include sense and antisense strands as appropriate to the goals of the therapy practiced according to the invention. Also, as used herein, "polynucleotide" refers to a polymer of deoxyribonucleotides or ribonucleotides, in the form of a separate fragment or as a component of a larger construct. Polynucleotides of the invention include functional derivatives of known polynucleotides which operatively encode for SERCA2a protein.

The polynucleotide sequence can be deduced from the genetic code, however, the degeneracy of the code must be taken into account. Polynucleotides useful in the invention include sequences which are degenerate as a result of the genetic code, which sequences may be readily determined by those of ordinary skill in the art.

Further, the term "heterologous" as it relates to nucleic acid sequences such as coding sequences and control sequences, denotes sequences that are not normally joined together, and/or are not normally associated with a particular cell. Thus, a "heterologous" region of a nucleic acid construct or a vector is a segment of nucleic acid within or attached to another nucleic acid molecule that is not found in association with the other molecule in nature. For example, a heterologous region of a nucleic acid construct could include a coding sequence flanked by sequences not found in association with the coding sequence in nature. Another example of a heterologous coding sequence is a construct where the coding sequence itself is not found in nature (*e*.*g*., synthetic sequences having codons different from the native gene). Similarly, a cell transformed with a construct which is not normally present in the cell would be considered heterologous for purposes of this invention. Allelic variation or naturally occurring mutational events do not give rise to heterologous DNA, as used herein.

Accordingly, a "coding sequence" or a sequence which "encodes" a particular protein, is a nucleic acid sequence which is transcribed (in the case of DNA) and translated (in the case of mRNA) into a polypeptide *in vitro* or *in vivo* when placed under the control of appropriate regulatory sequences. The boundaries of the coding sequence are determined by a start codon at the 5' (amino) terminus and a translation stop codon at the 3' (carboxy) terminus. A coding sequence can include, but is not limited to, cDNA from prokaryotic or eukaryotic mRNA, genomic DNA sequences from prokaryotic or eukaryotic DNA, and even synthetic DNA sequences. A transcription termination sequence will usually be located 3' to the coding sequence.

The nucleotide sequence of the SERCA2 and its isoforms is about 90%+ conserved among mammalian species. Hence, SERCA2 has been identified and sequenced from various mammalian species, including human GenBank sequences NM_170665 (Lytton and MacLennan, J Biol Chem (1998) 263(29):15024-15031); NM_001681 (Id.); NM_006241 (Park et al., J Biol Chem (1994) 269(2):944-954); NM_001003214 (Autry and Jones, J Biol Chem (1997) 272(25):15872-15880); BC035588 (Strausberg et al., Proc Natl Acad Sci USA (2002) 99(26):16899-16903); AY186578 (Gelebart et al., Biochem Biophys Res Comm (2003) 303(2):676-684); and mouse GenBank sequences NM_026482 (Du et al., Arch Biochem Biophys (1995) 316(1):302-310); NM_213616 (Hunter et al., Genomics (1993) 18(3):510-519); NM_009722 (Hsu et al., Biochem Biophys Res Comm (1993) 197(3):1483-1491); AJ131870 (Ver Heyen et al., Mamm Genome (2000) 11(2):159-163); BC054531 (Strausberg et al., Proc Natl Acad Sci USA (2002) 99(26):16899-16903); BC054748 (Id.); AJ131821(Ver Heyen et al., Mamm Genome (2000) 11(2):159-163); AJ223584 (Id.); AF029982 (Id.); and AF039893 (Schoenfeld and Lowe, direct submission (18-DEC-1997), Cardiovascular Research, Genentech, 1 DNA Way, South San Francisco, CA 94080, USA). All of the above sequences are publicly available.

Although those of ordinary skill in the art will recognize that use of the human SERCA2a polynucleotide would be preferred in human therapies, *e*.*g*., human gene therapies, rat SERCA2a polynucleotide can also be used for purposes of the invention described herein. Hence, polynucleotides which are structurally or functionally similar, *e*.*g*. highly homologous to human SERCA2a are encompassed within the invention.

It will be obvious to one skilled in the art, that to obtain and use SERCA2 sequences included in this disclosure and those known in the art, DNA and RNA may also be synthesized using automated nucleic acid synthesis by any method known now known or later discovered *(e.g.,* PCR, cDNA synthesis (see, for example, Sambrook et al., Molecular Cloning: A Laboratory Manual, 3rd Ed., Cold Spring Harbor Laboratory, Cold Spring Harbor, NY, 2001, and Current Protocols in Molecular Biology, M. Ausubel et al., eds., (Current Protocols, a joint venture between Greene Publishing Associates, Inc. and John Wiley & Sons, Inc., most recent Supplement)).

As will be understood by those skilled in the art, the polynucleotide compositions useful in this invention can include genomic sequences, extra-genomic and plasmid-encoded sequences and smaller engineered gene segments that express, or may be adapted to express, proteins, polypeptides, peptides and the like. Such segments may be naturally isolated, or modified synthetically by the hand of man.

As will be also recognized by the skilled artisan, polynucleotides useful in the invention may be single-stranded or double-stranded, and may be DNA (genomic, cDNA or synthetic) or RNA molecules. RNA molecules may include HnRNA molecules, which contain introns and correspond to a DNA molecule in a one-to-one manner, and mRNA molecules, which do not contain introns. Additional coding or noncoding sequences may, but need not, be present within a polynucleotide useful in the present invention, and a polynucleotide may, but need not, be linked to other molecules and/or support materials.

Polynucleotides may comprise a native sequence (*i*.*e*., an endogenous sequence that encodes a polypeptide/protein of the invention or a portion thereof) or may comprise a sequence that encodes a variant or derivative, including an immunogenic variant or derivative, of such a sequence. Further, polynucleotides can encode a combination of different sequences, *e*.*g*., a transgene, a viral polynucleotide, a nucleic acid encoding a selectable marker and the like.

As discussed previously, SERCA2 polypeptides are highly conserved, hence the skilled artisan can perform an optimal alignment of polypeptide or nucleic acid sequences for comparison using the Megalign program in the Lasergene suite of bioinformatics software (DNASTAR, Inc., Madison, Wis.), using default parameters. This program embodies several alignment schemes described in the following references: Dayhoff, M. O. (1978) A model of evolutionary change in proteins--Matrices for detecting distant relationships. In Dayhoff, M. O. (ed.) Atlas of Protein Sequence and Structure, National Biomedical Research Foundation, Washington D.C. Vol. 5, Suppl. 3, pp. 345-358; Hein J. (1990) Unified Approach to Alignment and Phylogenes pp. 626-645 Methods in Enzymology vol. 183, Academic Press, Inc., San Diego, Calif.; Higgins, D. G. and Sharp, P. M. (1989) CABIOS 5:151-153; Myers, E. W. and Muller W. (1988) CABIOS 4:11-17; Robinson, E. D. (1971) Comb. Theor 11:105; Saitou, N. Nei, M. (1987) Mol. Biol. Evol. 4:406-425; Sneath, P. H. A. and Sokal, R. R. (1973) Numerical Taxonomy--the Principles and Practice of Numerical Taxonomy, Freeman Press, San Francisco, Calif.; Wilbur, W. J. and Lipman, D. J. (1983) Proc. Natl. Acad., Sci. USA 80:726-730.

Alternatively, optimal alignment of sequences for comparison may be conducted by the local identity algorithm of Smith and Waterman (1981) Add. APL. Math 2:482, by the identity alignment algorithm of Needleman and Wunsch (1970) J. Mol. Biol. 48:443, by the search for similarity methods of Pearson and Lipman (1988) Proc. Natl. Acad. Sci. USA 85: 2444, by computerized implementations of these algorithms (GAP, BESTFIT, BLAST, FASTA, and TFASTA in the Wisconsin Genetics Software Package, Genetics Computer Group (GCG), 575 Science Dr., Madison, Wis.), or by inspection.

One example of algorithms that are suitable for determining percent sequence identity and sequence similarity are the BLAST and BLAST 2.0 algorithms, which are described in Altschul et al. (1977) Nucl. Acids Res. 25:3389-3402 and Altschul et al. (1990) J. Mol. Biol. 215:403-410, respectively. BLAST and BLAST 2.0 can be used, for example with the parameters described herein, to determine percent sequence identity for the polynucleotides and polypeptides disclosed herein. Software for performing BLAST analyses is publicly available through the National Center for Biotechnology Information. For amino acid sequences, a scoring matrix can be used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment.

In the compositions useful in the present invention, the polynucleotide or nucleic acid can be either a DNA or RNA. The sequences in question can be of natural or artificial origin, and in particular genomic DNA, cDNA, mRNA, tRNA, rRNA, hybrid sequences or synthetic or semi-synthetic sequences. In addition, the nucleic acid can be very variable in size, ranging from oligonucleotide to chromosome. These nucleic acids may be of human, animal, vegetable, bacterial, viral, and the like, origin. They may be obtained by any technique known to a person skilled in the art, and in particular by the screening of libraries, by chemical synthesis or alternatively by mixed methods including the chemical or enzymatic modification of sequences obtained by the screening of libraries. They can, moreover, be incorporated into vectors, such as plasmid vectors.

In order to express a desired polypeptide, the nucleotide sequences encoding the polypeptide, *e*.*g*., SERCA2, or functional equivalents, may be inserted into appropriate expression vector, *i.e.,* a vector which contains the necessary elements for the transcription and translation of the inserted coding sequence. Methods that are well known to those skilled in the art may be used to construct expression vectors containing sequences encoding a polypeptide of interest and appropriate transcriptional and translational control elements. These methods include *in vitro* recombinant DNA techniques, synthetic techniques, and *in vivo* genetic recombination. Such techniques are described, for example, in Sambrook, J. et al. (1989) Molecular Cloning: A Laboratory Manual, Cold Spring Harbor Press, Plainview, N.Y., and Ausubel, F. M. et al. (1989) Current Protocols in Molecular Biology, John Wiley & Sons, New York. N.Y.

The term "operatively encoding" refers to a polynucleotide which has been modified to include promoter and other sequences necessary for expression and, where desired, secretion of the desired translation product; *e*.*g*., a peptide or protein. All the embodiments of the invention can be practiced using known AAV expression vectors. Preferably, these vectors will include cDNA('s) which encode for the desired translation product. Therefore, unless context otherwise requires, it will be assumed that "polynucleotide" refers to operatively encoding sequences contained in a suitable recombinant expression vector, examples of which are provided herein.

The "control elements" or "regulatory sequences" present in an expression vector are those non-translated regions of the vector (*e*.*g*., enhancers, promoters, 5' and 3' untranslated regions and the like), which interact with host cellular proteins to carry out transcription and translation. Such elements may vary in their strength and specificity. Depending on the vector system and host utilized, any number of suitable transcription and translation elements, including constitutive and inducible promoters, may be used. For example, when cloning in bacterial systems, inducible promoters such as the hybrid lacZ promoter of the pBLUESCRIPT phagemid (Stratagene, La Jolla, Calif.) or pSPORTI plasmid (Gibco BRL, Gaithersburg, Md.) and the like may be used. In mammalian cell systems, promoters from mammalian genes or from mammalian viruses are generally preferred. By way of non-limiting examples, promoters include those from CMV, beta-actin, EF2alpha, RSV LTR, HIV LTR, HTLV-1 LTR, and composite promoters (D.H. Barouch et al, A human T-cell leukemia virus type 1 regulatory element enhances the immunogenicity of human immunodeficiency virus type 1 DNA vaccines in mice and nonhuman primates, J. Virol. 79: 8828-8834, 2005). In one aspect, the promoter is a CMV promoter or a promoter comprising portions of the chicken beta-actin promoter (H. Niwa et al, Efficient selection for high-expression transfectants with a novel eukaryotic vector, Gene 108:193-199, 1991). In another aspect, the promoter is a CMV promoter, Sm22 promoter, or Tie2 promoter. If it is necessary to generate a cell line that contains multiple copies of the sequence encoding a polypeptide, vectors based on SV40 or EBV may be advantageously used with an appropriate selectable marker.

Specific initiation signals may also be used to achieve more efficient translation of sequences encoding a polypeptide of interest. Such signals include the ATG initiation codon and adjacent sequences. In cases where sequences encoding the polypeptide, its initiation codon, and upstream sequences are inserted into the appropriate expression vector, no additional transcriptional or translational control signals may be needed. However, in cases where only coding sequence, or a portion thereof, is inserted, exogenous translational control signals including the ATG initiation codon should be provided. Furthermore, the initiation codon should be in the correct reading frame to ensure translation of the entire insert. Exogenous translational elements and initiation codons may be of various origins, both natural and synthetic. The efficiency of expression may be enhanced by the inclusion of enhancers which are appropriate for the particular cell system which is used, such as those described in the literature (Scharf, D. et al. (1994) Results Probl. Cell Differ. 20:125-162).

In addition, a host cell strain may be chosen for its ability to modulate the expression of the inserted sequences or to process the expressed protein in the desired fashion. Such modifications of the polypeptide include, but are not limited to, acetylation, carboxylation, glycosylation, phosphorylation, lipidation, and acylation. Post-translational processing which cleaves a "prepro" form of the protein may also be used to facilitate correct insertion, folding and/or function. Different host cells such as CHO, COS, HeLa, MDCK, HEK293, and WI38, which have specific cellular machinery and characteristic mechanisms for such post-translational activities, may be chosen to ensure the correct modification and processing of the foreign protein.

Moreover, gene delivery involves polymers which form complexes, nanoparticles (defined as less than 1 micron in diameter), or even microparticles (defined as 1 micron in diameter or greater) with DNA plasmids and other nucleic acids. Many kinds of polymers have been described that enhance the expression of genes encoded by nucleic acids in cells.

For example, cationic polymers such as poly-L-lysine, poly-L-glutamate, or block co-polymers may also be delivery agents for nucleic acids. Further, the use of poly[alpha-(4-aminobutyl)-1-glycolic acid] (PAGA) has been used to deliver plasmid DNAs to tumor-bearing mice. Still, the use of water-soluble lipopolymer (WSLP), using branched polyethylenimine and cholesteryl chloroformate has also been described. Polyethylenimine-based vesicle-polymer hybrid gene delivery as another way to deliver plasmid DNA expression vectors, including the use of poly(propylenimine) dendrimers as delivery agents Also, polyethylene glycol (PEG) copolymers were found to improve plasmid DNA delivery, including various kinds of polymers that can be used for the controlled release of plasmid DNA and other nucleic acids. Such molecules include poly(lactic acid) and its derivatives, PEGylated poly(lactic acid), poly(lactic-co-glycolic acid) and its derivatives, poly(ortho esters) and their derivatives, PEGylated poly(ortho esters), poly(caprolactone) and its derivatives, PEGylated poly(caprolactone), polylysine and its derivatives, PEGylated polylysine, poly(ethylene imine) and its derivatives, PEGylated poly(ethylene imine), poly(acrylic acid) and its derivatives, PEGylated poly(acrylic acid), poly(urethane) and its derivatives, PEGylated poly(urethane), and combinations of all of these. The use of polymeric lipid-protein-sugar microparticles for the delivery of nucleic acids is disclosed herein. These and other polymers are well known in the art.

Nucleic acid compositions may be delivered by electroporation. Elecroporation uses electrical pulses to introduce proteins, nucleic acids, lipids, carbohydrates, or mixtures thereof into the host to produce an effect. A typical use of electroporation is to introduce a nucleic acid into the host so that the protein encoded by the nucleic acid is efficiently produced.

Nucleic acid compositions may be delivered by particle bombardment. Powderject (Norvartis Pharmaceutical Corporation) has developed methods to coat gold particles with nucleic acids and other substances and then forcibly introduce them into the host by particle bombardment. For nucleic acids encoding antigens, this results in an improved immune response to the antigens.

The present invention also embodies administering "pharmaceutically acceptable" molecular entities and compositions that are physiologically tolerable and do not typically produce an allergic or similar untoward reaction, such as gastric upset, dizziness and the like, when administered to a human. Thus, as used herein, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E. W. Martin.

Accordingly, as used herein, the term "therapeutic composition" is defined as one comprising at least a gene encoding SERCAisoform 2a (SERCA2a). The therapeutic composition may also contain other nucleic acid molecules (*e*.*g*., a viral vector) and pharmaceutically acceptable entities, and substances such as water, minerals, carriers such as proteins, and other excipients known to one skilled in the art.

The invention relates to treating or providing treatment for a subject suffering from pulmonary hypertension. As used herein, "treating" or "treatment" of the subject is an approach for obtaining beneficial or desired clinical results. Desired clinical results include, but are not limited to, prevention of remodeling of smooth muscle cells on pulmonary arteries, restored lung function, and/or reduction of severity of or inhibition of symptoms associated with pulmonary hypertension. Exemplary symptoms associated with pulmonary hypertension include, but are not limited to, chest pain, dizziness, fainting, fatigue, leg swelling, light-headedness during exercise or exertion, shortness of breath during activity, and weakness. The desired benefits or clinical results are independent of the mechanism, although the mechanism of activity is encompassed within the result. As will be understood by one of skill in the art, the particular symptoms which yield to treatment in accordance with the invention will depend on severity of the pulmonary hypertension being treated.

In one aspect of the invention, the therapeutic composition used to treat pulmonary hypertension includes an AAV expression vector or AAV virion and a gene encoding SERCA isoform 2a. As previously discussed, the SERCA2a transgene of the invention, when contained in a therapeutic composition, is typically operatively linked to various regulatory elements which has been modified to include promoter and other sequences necessary for expression and, where desired, secretion of the desired translation product (*e*.*g*., a peptide or protein). For example, the SERCA2a polynucleotides may be conjugated to or used in association with other polynucleotides which operatively code for regulatory proteins that control the expression of these polypeptides or may contain recognition, promoter and secretion sequences. Those of ordinary skill in the art will be able to select regulatory polynucleotides and incorporate them into SERCA2a polynucleotides of the invention without undue experimentation.

A therapeutic composition to treat or reduce symptoms associated with pulmonary hypertension may include a polynucleotide encoding a protein capable of indirectly modulating smooth muscle Ca²⁺ and contractility. As is known in the art, the cardiac protein phospholamban is inhibitory to the activity of SERCA2a. Accordingly, disclosed herein are methods to decrease the level or activity of phospholamban in a pulmonary smooth muscle cell. In one embodiment, expression of a pseudophosphorylated mutant of phospholamban is increased. In another embodiment, a mutant has replacement of the serine 16 phosphorylation site with the basic amino acid glutamine, thereby introducing a negative charge at position 16 (S16E phospholamban mutant). This pseudophosphorylated form of phospholamban competes with natural phospholamban for binding to SERCA, thereby decreasing the opportunity for the natural protein to negatively affect SERCA activity. See, *e.g.,* WO 2000/025804.

Disclosed herein are methods that include administering to a subject in need thereof a therapeutically effective amount of an siRNA in a viral vector, where the RNAi decreases expression and/or activity of phospholamban (PLB), in an amount effective to transduce pulmonary smooth muscle cells of the subject, thereby resulting in expression of the RNAi and treating pulmonary hypertension in the subject. Unphosphorylated PLB keeps the Ca²⁺ affinity of SERCA2a low, resulting in decreased SR Ca²⁺ uptake, slowed relaxation and decreased SR Ca²⁺ load.

PLB nucleic acids, include but are not limited to, GenBank Accession Nos. NM_02667 (Simmerman et al., J Biol Chem (1986) 261(28):13333-13341); NM_023129 (Ganim et al., Cir Res (1992) 71(5):1021-1030); NM_022707 (Wang and Nadal-Ginard, Adv Exp Med Biol (1991) 304:387-395); BC134584 (Moore et al., direct submission (17-MAR-2007), BC Cancer Agency, Canada's Michael Smith Genome Sciences Centre, Suite 100, 570 West 7th Avenue, Vancouver, British Columbia V5Z 4S6, Canada); and NM_214213 (Verboomen et al., Biochem J (1989) 262(1):353-356). All of the above sequences are publicly available.

The term "RNA interference" refers generally to a process in which a double-stranded RNA molecule changes the expression of a nucleic acid sequence with which the double-stranded or short hairpin RNA molecule shares substantial or total homology. While not being bound by theory, the mechanism of action may include, but is not limited to, direct or indirect down regulation of the expression of the PLB gene, decrease in PLB mRNA, and/or a decrease in PLB activity. The term "RNAi," including "short inhibitory RNA (siRNA)," refer to RNA sequences that elicit RNA interference, and which is transcribed from a vector. The terms "short hairpin RNA" or "shRNA" refer to an RNA structure having a duplex region and a loop region. This term should also be understood to specifically include RNA molecules with stem-loop or panhandle secondary structures. RNAis may be expressed initially as shRNAs.

RNAi is generally optimised by identical sequences between the target and the RNAi. The RNA interference phenomenon can be observed with less than 100% homology, but the complementary regions must be sufficiently homologous to each other to form the specific double stranded regions. The precise structural rules to achieve a double-stranded region effective to result in RNA interference have not been fully identified, but approximately 70% identity is generally sufficient. Accordingly, in some embodiments of the invention, the homology between the RNAi and PLB is at least 70% nucleotide sequence identity, and may be at least 75% nucleotide sequence identity. Homology includes, but is not limited to, at least 80% nucleotide sequence identity, and is at least 85% or even 90% nucleotide sequence identity. In one embodiment, sequence homology between the target sequence and the sense strand of the RNAi is at least 90%, 91%, 92%, 93%, 94%, 95%, 96%, 97%, 98%, 99% or 100% nucleotide sequence identity.

Another consideration is that base-pairing in RNA is subtly different from DNA in that G will pair with U, although not as strongly as it does with C, in RNA duplexes. Moreover, for RNAi efficacy, it is more important that the antisense strand be homologous to the target sequence. In some circumstances, it is known that 17 out of 21 nucleotides is sufficient to initiate RNAi, but in other circumstances, identity of 19 or 20 nucleotides out of 21 is required. While not being bound by theory, at a general level, greater homology is required in the central part of a double stranded region than at its ends. Some predetermined degree of lack of perfect homology may be designed into a particular construct so as to reduce its RNAi activity which would result in a partial silencing or repression of the target gene's product, in circumstances in which only a degree of silencing was sought. In such a case, only one or two bases of the antisense sequence may be changed. On the other hand, the sense strand is more tolerant of mutations. While not being bound by theory, this may be due to the antisense strand being the one that is catalytically active. Thus, less identity between the sense strand and the transcript of a region of a target gene will not necessarily reduce RNAi activity, particularly where the antisense strand perfectly hybridizes with that transcript. Mutations in the sense strand (such that it is not identical to the transcript of the region of the target gene) may be useful to assist sequencing of hairpin constructs and potentially for other purposes, such as modulating dicer processing of a hairpin transcript or other aspects of the RNAi pathway.

The terms "hybridizing" and "annealing" including grammatical equivalents thereof, are used interchangeably in this specification with respect to nucleotide sequences and refer to nucleotide sequences that are capable of forming Watson-Crick base pairs due to their complementarity. The person skilled in the art would understand that non-Watson-Crick base-pairing is also possible, especially in the context of RNA sequences. For example a so-called "wobble pair" can form between guanosine and uracil residues in RNA.

The RNA expression products of the RNAi expression cassette lead to the generation of a double-stranded RNA (dsRNA) complex for inducing RNA interference and thus down-regulating or decreasing expression of a mammalian gene. "dsRNA" refers to a ribonucleic acid complex comprising two Watson-Crick base-paired complementary RNA strands. The dsRNA complex comprises a first nucleotide sequence that hybridizes under stringent conditions, including a wash step of 0.2 x SSC at 65°C, to a nucleotide sequence of at least one mammalian gene and a second nucleotide sequence which is complementary to the first nucleotide sequence. The first nucleotide sequence might be linked to the second nucleotide sequence by a third nucleotide sequence (*e*.*g*., an RNA loop) so that the first nucleotide sequence and the second nucleotide sequence are part of the same RNA molecule; alternatively, the first nucleotide sequence might be part of one RNA molecule and the second nucleotide sequence might be part of another RNA molecule. Thus, a dsRNA complex may be formed by intramolecular hybridization or annealing or the ds RNA complex is formed by intermolecular hybridization or annealing.

Still other nucleic acids (transgenes) and proteins of SERCA, phospholamban, inhibitor-1 of the type 1 phosphatase, S100A1, and sarcolipin, as well as related nucleic acids and proteins which play a role in Ca2+, are targets for polynucleotides disclosed herein.

For example, in one aspect of the invention, a viral vector and transgene is AAV2/1/SERCA2a, which is comprised of an AAV serotype 1 viral capsid enclosing a single-stranded 4486 nucleotide DNA containing the human SERCA2a expression cassette flanked by ITRs derived from AAV serotype 2. The icosahedral capsid consists of three related AAV serotype 1 capsid proteins, VP1, VP2, and VP3. The AAV2/1/SERCA2a DNA contains the following components: AAV serotype2 based ITR at the 3' and 5' ends, flanking the CMV-hSERCA2a-polyA expression cassette. The expression cassette contains the cytomegalovirus immediate early enhancer/promoter (CMVie) driving transcription of sequences including a hybrid intron from the commercial plasmid pCI (Promega - GenBank U47119), the hSERCA2A cDNA (coding sequence identical to GenBank NM-001681), and a bovine growth hormone polyadenylation signal (BGHpA, (GenBank M57764)). The hybrid intron was designed using the 5'-donor site from the first intron of the human β-globin and 3'-acceptor site from the intron located between the leader and body of an immunoglobulin gene heavy chain variable region.

The AAV2/1/SERCA2a vector or construct incorporates less than 300 nucleotides of the wild-type AAV (wtAAV) sequences in the vector genome. The wtAAV sequences are AAV serotype 2 derived ITRs that provide in cis the packaging signal that allows the SERCA2a DNA to be inserted into the capsid.

The therapeutic agents, including polynucleotides, polynucleotides in combination with a vector, both viral and non-viral, as discussed above can be used in the preparation of a medicament for the treatment of pulmonary disease (*e*.*g*., pulmonary hypertension), where the medicament is administered by direct infusion into the circulatory system or by intra-tracheal or inhalation administration. Methods for delivery via inhalation are discussed in, *e*.*g*., Moss, et al. Human Gene Therapy, 18:726-732 (August 2007).

The therapeutic compositions useful in the present invention are delivered to a "target cell" or "host cell" or "target tissue" and equivalents thereof, which refers to the cell, tissue of the host in which expression of the operatively encoding polynucleotide is sought. For example, adeno-associated viral-SERCA2 compositions are delivered in a muscle cell or muscle tissue.

Disclosed herein is packaging of the AAV-SERCA2 virions as discussed previously. Growth and propagation of the virions will require a "defined-medium conditions", which refer to environments for culturing cells where the concentration of components therein required for optimal growth are detailed. For example, depending on the use of the cells (*e*.*g*., therapeutic applications), removing cells from conditions that contain xenogenic proteins are important; *i.e.,* the culture conditions are typically animal-free conditions or free of non-human animal proteins.

The present invention provides AAV expression vectors or rAAV virions for use in treating "pulmonary hypertension," which refers generally to abnormally high blood pressure in the arteries of the lungs. It makes the right side of the heart need to work harder than normal.

Pulmonary hypertension, as described herein, is commonly diagnosed using a variety of methods, including a physical examination performed to look for typical signs of pulmonary hypertension. In this examination a physician looks for altered heart sounds, such as a widely split S₂ or second heart sound, a loud P₂ or pulmonic valve closure sound (part of the second heart sound), (para)sternal heave, possible S₃ or third heart sound, and pulmonary regurgitation. Further procedures are required to confirm the presence of pulmonary hypertension and exclude other possible diagnoses. These generally include pulmonary function tests; blood tests to exclude HIV, autoimmune diseases, and liver disease; electrocardiography (ECG); arterial blood gas measurements; X-rays of the chest (followed by high-resolution CT scanning if interstitial lung disease is suspected); and ventilation-perfusion or V/Q scanning to exclude chronic thromboembolic pulmonary hypertension. Biopsy of the lung is usually not indicated unless the pulmonary hypertension is thought to be due to an underlying interstitial lung disease. But lung biopsies are fraught with risks of bleeding due to the high intrapulmonary blood pressure. Clinical improvement is often measured by a "six-minute walk test," *i.e.,* the distance a patient can walk in six minutes. Stability and improvement in this measurement correlate with better survival. Blood BNP level is also being used now to follow progress of patients with pulmonary hypertension.

In a related aspect, the therapeutic compositions of the present invention are administered to a subject as a ameliorative modality. As used herein, "ameliorative," means to improve or relieve a subject of symptoms associated with a disorder, and includes curing such a disorder.

It will be understood that, if desired, a composition as disclosed herein may be administered in combination with other agents as well, such as, *e.g.,* other proteins or polypeptides or various pharmaceutically-active agents. In fact, there is virtually no limit to other components that may also be included, given that the additional agents do not cause a significant adverse effect upon contact with the target cells or host tissues. The compositions may thus be delivered along with various other agents as required in the particular instance. Such compositions may be purified from host cells or other biological sources, or alternatively may be chemically synthesized as described herein. Likewise, such compositions may further comprise substituted or derivatized RNA or DNA compositions.

It will be apparent that any of the pharmaceutical compositions described herein can contain pharmaceutically acceptable salts of the polynucleotides and polypeptides of the invention. Such salts can be prepared, for example, from pharmaceutically acceptable non-toxic bases, including organic bases (*e*.*g*., salts of primary, secondary and tertiary amines and basic amino acids) and inorganic bases (*e*.*g*., sodium, potassium, lithium, ammonium, calcium and magnesium salts).

Embodiments describing adeno-associated viral systems and expression vectors were previously described. However, the term "vector" means any genetic element, such as a plasmid, phage, transposon, cosmid, chromosome, virus, virion, etc., which is capable of replication when associated with the proper control elements and which can transfer gene sequences between cells. Thus, the term includes cloning and expression vehicles, as well as viral vectors previously discussed. Similarly, "recombinant expression vector" refers to systems of polynucleotide(s) which operatively encode polypeptides expressible in eukaryotes or prokaryotes. Methods of expressing DNA sequences having eukaryotic or viral sequences in prokaryotes are well known in the art. Biologically functional viral and plasmid DNA vectors capable of expression and replication in a host are also well known in the art. Hosts can include microbial, yeast, insect and mammalian organisms.

The vectors or recombinant expression vectors provided herein are easily manufactured, and combine the advantages of adenovirus (high titer, high infectivity, large capacity, lack of association with human malignancy) but with the integration capability of AAV, making them particularly suitable for stable gene transfer which is useful in, for example, gene therapy approaches such as that described herein. A further advantage of the described AAV vectors is that, by virtue of containing AAV TR or ITR and D sequences that flank the gene of interest, it is expected that they integrate into cellular chromosomal DNA. Integration is important for stable gene transfer into cells. Thus, the AAV vectors described herein are preferred over, for example, adenovirus vectors, since they are episomal and would be lost after several cell divisions. Another advantage of the AAV vectors provided herein is that they are packaged efficiently into stable virus particles whether small or large polynucleotides are used. Still, another advantage of the AAV vectors provided herein is that they are less cytotoxic than first generation adenovirus vectors since no adenovirus genes are expressed within transduced cells.

The AAV-vector and/or AAV-SERCA2 containing virions described herein are "transfected", which refers to the uptake of foreign DNA by a cell. That is, a cell "transfected" with exogenous DNA and the DNA is introduced inside the cell membrane. A number of transfection techniques are generally known in the art. See, *e.g.,* Graham et al. (1973) Virology, 52:456, Sambrook et al. (1989) Molecular Cloning, a laboratory manual, Cold Spring Harbor Laboratories, New York, Davis et al. (1986) Basic Methods in Molecular Biology, Elsevier, and Chu et al. (1981) Gene 13:197. Such techniques can be used to introduce one or more exogenous DNA moieties, such as a nucleotide integration vector and other nucleic acid molecules, into suitable host cells.

The term "host cell" or "host" denotes, for example, mammalian cells, that can be, or have been, used as recipients of an AAV helper construct, an AAV vector plasmid, an accessory function vector, or other transfer DNA. Similarly, the terms "subject", "individual" or "patient" are used interchangeably herein and refer to a vertebrate, preferably a mammal. Mammals include, but are not limited to, murines, simians, humans, farm animals, sport animals and pets. The term includes the progeny of the original cell which has been transfected. Thus, a "host cell" or "host" as used herein generally refers to a cell which has been transfected with an exogenous DNA sequence. It is understood that the progeny of a single parental cell may not necessarily be completely identical in morphology or in genomic or total DNA complement as the original parent, due to natural, accidental, or deliberate mutation.

As used herein, the term "cell line" refers to a population of cells capable of continuous or prolonged growth and division *in vitro.* Often, cell lines are clonal populations derived from a single progenitor cell. It is further known in the art that spontaneous or induced changes can occur in karyotype during storage or transfer of such clonal populations. Therefore, cells derived from the cell line referred to may not be precisely identical to the ancestral cells or cultures, and the cell line referred to includes such variants.

Additional viral vectors useful for delivering the polynucleotides encoding polypeptides disclosed herein by gene transfer include those derived from the pox family of viruses, such as vaccinia virus and avian poxvirus. By way of example, vaccinia virus recombinants expressing the novel molecules can be constructed as follows. The DNA encoding a polypeptide is first inserted into an appropriate vector so that it is adjacent to a vaccinia promoter and flanking vaccinia DNA sequences, such as the sequence encoding thymidine kinase (TK). This vector is then used to transfect cells which are simultaneously infected with vaccinia. Homologous recombination serves to insert the vaccinia promoter plus the gene encoding the polypeptide of interest into the viral genome. The resulting TK⁽⁻⁾ recombinant can be selected by culturing the cells in the presence of 5-bromodeoxyuridine and picking viral plaques resistant thereto.

A vaccinia-based infection/transfection system can be conveniently used to provide for inducible, transient expression or coexpression of one or more polypeptides described herein in host cells of an organism. In this particular system, cells are first infected *in vitro* with a vaccinia virus recombinant that encodes the bacteriophage T7 RNA polymerase. This polymerase displays exquisite specificity in that it only transcribes templates bearing T7 promoters. Following infection, cells are transfected with the polynucleotide or polynucleotides of interest, driven by a T7 promoter. The polymerase expressed in the cytoplasm from the vaccinia virus recombinant transcribes the transfected DNA into RNA which is then translated into polypeptide by the host translational machinery. The method provides for high level, transient, cytoplasmic production of large quantities of RNA and its translation products. See, *e*.*g*., Elroy-Stein and Moss, Proc. Natl. Acad. Sci. USA (1990) 87:6743-6747; Fuerst et al. Proc. Natl. Acad. Sci. USA (1986) 83:8122-8126.

Alternatively, avipoxviruses, such as the fowlpox and canarypox viruses, can also be used to deliver the coding sequences of interest. Recombinant avipox viruses, expressing immunogens from mammalian pathogens, are known to confer protective immunity when administered to non-avian species. The use of an Avipox vector is particularly desirable in human and other mammalian species since members of the Avipox genus can only productively replicate in susceptible avian species and therefore are not infective in mammalian cells. Methods for producing recombinant Avipoxviruses are known in the art and employ genetic recombination, as described above with respect to the production of vaccinia viruses. See, *e*.*g*., WO 91/12882; WO 89/03429; and WO 92/03545.

Any of a number of alphavirus vectors can also be used for delivery of polynucleotide compositions of the present invention, such as those vectors described in U.S. Pat. Nos. 5,843,723; 6,015,686; 6,008,035 and 6,015,694. Certain vectors based on Venezuelan Equine Encephalitis (VEE) can also be used, illustrative examples of which can be found in U.S. Pat. Nos. 5,505,947 and 5,643,576.

Moreover, molecular conjugate vectors, such as the adenovirus chimeric vectors described in Michael et al. J. Biol. Chem. (1993) 268:6866-6869 and Wagner et al. Proc. Natl. Acad. Sci. USA (1992) 89:6099-6103, can also be used for gene delivery under the invention.

In certain embodiments, a polynucleotide may be integrated into the genome of a target cell. This integration may be in the specific location and orientation via homologous recombination (gene replacement) or it may be integrated in a random, non-specific location (gene augmentation). In yet further embodiments, the polynucleotide may be stably maintained in the cell as a separate, episomal segment of DNA. Such polynucleotide segments or "episomes" encode sequences sufficient to permit maintenance and replication independent of or in synchronization with the host cell cycle. The manner in which the expression construct is delivered to a cell and where in the cell the polynucleotide remains is dependent on the type of expression construct employed.

In a related embodiment, other devices and methods that may be useful for gas-driven needle-less injection of compositions of the present invention include those provided by Bioject, Inc. (Portland, Oreg.), some examples of which are described in U.S. Pat. Nos. 4,790,824; 5,064,413; 5,312,335; 5,383,851; 5,399,163; 5,520,639 and 5,993,412.

The present invention may be delivered by various modes. For oral administration the compositions of the present invention may alternatively be incorporated with one or more excipients in the form of a mouthwash, dentifrice, buccal tablet, oral spray, or sublingual orally-administered formulation. Alternatively, the active ingredient may be incorporated into an oral solution such as one containing sodium borate, glycerin and potassium bicarbonate, or dispersed in a dentifrice, or added in a therapeutically-effective amount to a composition that may include water, binders, abrasives, flavoring agents, foaming agents, and humectants. Alternatively the compositions may be fashioned into a tablet or solution form that may be placed under the tongue or otherwise dissolved in the mouth. Vaccine formulations can also be delivered to the nasal mucosa, aerosolized for inhalational delivery, or delivered to the mucosal surfaces of the female and male genital track or the rectum. Vaccine formations may also be formulated for transdermal delivery.

In certain circumstances it will be desirable to deliver the pharmaceutical compositions disclosed herein parenterally, intravenously, intramuscularly, intratracheally, or even intraperitoneally. Such approaches are well known to the skilled artisan, some of which are further described, for example, in U.S. Pat. No. 5,543,158; U.S. Pat. No. 5,641,515 and U.S. Pat. No. 5,399,363. As such, in one embodiment, the compositions of the invention may be delivered via inhalation, intravenous injection, intracardiac injection, intratracheally. In another embodiment, the compositions of the invention are administered via mechanical ventilation. In certain embodiments, solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations generally will contain a preservative to prevent the growth of microorganisms.

Illustrative pharmaceutical forms suitable for injectable use include sterile aqueous solutions or dispersions and sterile powders for the extemporaneous preparation of sterile injectable solutions or dispersions (for example, see U.S. Pat. No. 5,466,468). In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (*e*.*g*., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and/or by the use of surfactants. The prevention of the action of microorganisms can be facilitated by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

In one embodiment, for parenteral administration in an aqueous solution, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, a sterile aqueous medium that can be employed will be known to those of skill in the art in light of the present disclosure. For example, one dosage may be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. Moreover, for human administration, preparations will of course preferably meet sterility, pyrogenicity, and the general safety and purity standards as required by FDA Office of Biologics standards.

The compositions disclosed herein may be formulated in a neutral or salt form. Illustrative pharmaceutically-acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. Upon formulation, solutions will be administered in a manner compatible with the dosage formulation and in such amount as is therapeutically effective.

The carriers can further comprise any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions. The phrase "pharmaceutically-acceptable" refers to molecular entities and compositions that do not produce an allergic or similar untoward reaction when administered to a human.

In certain embodiments, the pharmaceutical compositions may be delivered by intranasal sprays, inhalation, and/or other aerosol delivery vehicles. Methods for delivering genes, nucleic acids, and peptide compositions directly to the lungs via nasal aerosol sprays has been described, e.g., in U.S. Pat. No. 5,756,353 and U.S. Pat. No. 5,804,212. Likewise, the delivery of polynucleotides (*e*.*g*., viral vectors) using intranasal microparticle resins (Takenaga et al., J Controlled Release (1998) 52(1-2):81-7; and Moss, et al. Human Gene Therapy, 18:726-732 (August 2007)) and lysophosphatidyl-glycerol compounds (U.S. Pat. No. 5,725,871) are also well-known in the pharmaceutical arts. Likewise, illustrative transmucosal drug delivery in the form of a polytetrafluoroetheylene support matrix is described in U.S. Pat. No. 5,780,045.

In certain embodiments, liposomes, nanocapsules, microparticles, lipid particles, vesicles, and the like, are used for the introduction of the vectors/virions of the present invention into suitable host cells/organisms. In particular, the vectors/virions of the present invention may be formulated for delivery either encapsulated in a lipid particle, a liposome, a vesicle, a nanosphere, or a nanoparticle or the like. Alternatively, vectors/virions of the present invention can be bound, either covalently or non-covalently, to the surface of such carrier vehicles.

The formation and use of liposome and liposome-like preparations as potential drug carriers is generally known to those of skill in the art (see for example, Lasic, Trends Biotechnol (1998) 16(7):307-21; Takakura, Nippon Rinsho (1998) 56(3):691-5; Chandran et al., Indian J Exp Biol (1997) 35(8):801-9; Margalit, Crit Rev Ther Drug Carrier Syst (1995) 12(2-3):233-61; U.S. Pat. No. 5,567,434; U.S. Pat. No. 5,552,157; U.S. Pat. No. 5,565,213; U.S. Pat. No. 5,738,868 and U.S. Pat. No. 5,795,587.

Alternatively, disclosed herein are pharmaceutically-acceptable nanocapsule formulations of the compositions sdisclosed herein. Nanocapsules can generally entrap compounds in a stable and reproducible way (see, for example, Quintanar-Guerrero et al., Drug Dev India Pharm (1998) 24(12):1113-28). To avoid side effects due to intracellular polymeric overloading, such ultrafine particles (sized around 0.1 µm) may be designed using polymers able to be degraded *in vivo.* Such particles can be made as described, for example, by Couvreur et al., Crit Rev Ther Drug Carrier Syst. 1988; 5(1):1-20; zur Muhlen et al., Eur J Pharm Biopharm (1998) 45(2):149-55; Zambaux et al., J Controlled Release (1998) 50(1-3):31-40; and U.S. Pat. No. 5,145,684.

The present invention contemplates a variety of dosing schedules described herein as well as others not described herein but would otherwise be known to one skilled in the art. The invention encompasses continuous dosing schedules, in which a vector or virion of the invention is administered on a regular (daily, weekly, or monthly, depending on the dose and dosage form) basis without substantial breaks. Preferred continuous dosing schedules include daily continuous administration where a vector or virion of the invention is administered each day, and continuous bolus administration schedules, where a vector or virion of the is administered at least once per day by intravenous or subcutaneous injections. Exemplary continuous administration schedules include, but are not limited to, at least 2, 3, 4, 5, 6, or 7 days, at least 1, 2, 3, 4, 5, or 6 weeks or more, or any combination thereof.

The invention also encompasses discontinuous dosing schedules. The exact parameters of discontinuous administration schedules will vary according the formulation, method of delivery and the clinical needs of the subject. For example, if the a vector or virion of the invention is administered by inhalation, administration schedules comprising a first period of administration followed by a second period in which the vector or virion of the invention is not administered which is greater than, equal to, or less than the period where the vector or virion of the invention is administered. Examples of discontinuous administration schedules for inhalation administration include, but are not limited to, schedules comprising periods selected from 1, 2, 3, 4, 5, 6, or 7 days, 1, 2, 3, 4, 5, 6, or more weeks, or any combination thereof, and off periods selected from 1, 2, 3, 4, 5, 6, or 7 days, 1, 2, 3, 4, 5, 6 or more weeks.

Continuous and discontinuous administration schedules by any method also include dosing schedules in which the dose is modulated throughout the effective period, such that, for example, at the beginning of the vector or virion of the invention administration period; the dose is low and increased until the end of the vector or virion of the invention administration period; the dose is initially high and decreased during the vector or virion of the invention administration period; the dose is initially low, increased to a peak level, then reduced towards the end of the vector or virion of the invention administration period; and any combination thereof. Also, the dosing schedules may be performed using any method of standard in the art, such as a catheter system.

Compositions will comprise sufficient genetic material to produce a therapeutically effective amount of the SERCA2 or portions or fragments or functional fragments of interest, *i*.*e*., an amount sufficient to reduce or ameliorate symptoms of the disease state in question or an amount sufficient to confer the desired benefit. The compositions may also contain a pharmaceutically acceptable excipient. Such excipients include any pharmaceutical agent that does not itself induce the production of antibodies harmful to the individual receiving the composition, and which may be administered without undue toxicity. Pharmaceutically acceptable excipients include, but are not limited to, sorbitol, any of the various TWEEN compounds, and liquids such as water, saline, glycerol and ethanol. Pharmaceutically acceptable salts can be included therein, for example, mineral acid salts such as hydrochlorides, hydrobromides, phosphates, sulfates, and the like; and the salts of organic acids such as acetates, propionates, malonates, benzoates, and the like. Additionally, auxiliary substances, such as wetting or emulsifying agents, pH buffering substances, and the like, may be present in such vehicles. A thorough discussion of pharmaceutically acceptable excipients is available in REMINGTON'S PHARMACEUTICAL SCIENCES (Mack Pub. Co., N.J. 1991).

One particularly useful formulation includes recombinant AAV virions in combination with one or more dihydric or polyhydric alcohols, and, optionally, a detergent, such as a sorbitan ester. See, for example, International Publication No. WO 00/32233.

As is apparent to those skilled in the art in view of the teachings of this specification, an effective amount of viral vector which must be added can be empirically determined. Administration can be effected in one dose, continuously or intermittently throughout the course of treatment. Methods of determining the most effective means and dosages of administration are well known to those of skill in the art and will vary with the viral vector, the composition of the therapy, the target cells, and the subject being treated. Single and multiple administrations can be carried out with the dose level and pattern being selected by the treating physician.

It should be understood that more than one transgene can be expressed by the delivered recombinant virion. Alternatively, separate vectors, each expressing one or more different transgenes, can also be delivered as described herein. Furthermore, it is also intended that the viral vectors delivered by the methods of the present invention be combined with other suitable compositions and therapies. Where the transgene is under the control of an inducible promoter, certain systemically delivered compounds such as muristerone, ponasteron, tetracyline or aufin may be administered in order to regulate expression of the transgene.

Accordingly, therapeutic compositions of the present invention may contain the SERC2a transgene operatively linked to various regulatory elements in an AAV vector, substantially similar to that previously described above including any excipients or carriers or agents necessary to effectuate efficient, but non-toxic delivery of the therapeutic compositions will be produced. Also control AAV compositions will be produced, e.g. AAV-GFP constructs.

Also, in order to distinguish AAV-delivered SERCA2a from its endogenous counterpart, an AAV vector can be constructed which encodes a recombinant fusion SERCA2a operatively linked to green fluorescent protein (GFP) tag (AAV-SERCA2a-GFP), for recognition.

The host cell (or packaging cell) must also be rendered capable of providing nonAAV-derived functions, or "accessory functions", in order to produce rAAV virions. Accessory functions are nonAAV-derived viral and/or cellular functions upon which AAV is dependent for its replication, including at least those nonAAV proteins and RNAs that are required in AAV replication, including those involved in activation of AAV gene transcription, stage specific AAV mRNA splicing, AAV DNA replication, synthesis of Cap expression products and AAV capsid assembly. Viral-based accessory functions can be derived from any of the known helper viruses.

In particular, accessory functions can be introduced into and then expressed in host cells using methods known to those of skill in the art. Typically, accessory functions are provided by infection of the host cells with an unrelated helper virus. A number of suitable helper viruses are known, including adenoviruses; herpesviruses, *e*.*g*., herpes simplex virus types 1 and 2; and vaccinia viruses. Nonviral accessory functions will also find use herein, such as those provided by cell synchronization using any of various known agents. See, *e*.*g*., Buller et al. (1981) J. Virol. 40:241 247; McPherson et al. (1985) Virology 147:217 222; Schlehofer et al. (1986) Virology 152:110 117.

Alternatively, accessory functions can be provided using an accessory function vector as defined above. See, *e.g.,* U.S. Pat. No. 6,004,797 and International Publication No. WO 01/83797. Nucleic acid sequences providing the accessory functions can be obtained from natural sources, such as from the genome of an adenovirus particle, or constructed using recombinant or synthetic methods known in the art. Also, the full-complement of adenovirus genes is not required for accessory helper functions. In fact, adenovirus mutants incapable of DNA replication and late gene synthesis have been shown to be permissive for AAV replication. Ito et al., (1970) J. Gen. Virol. 9:243; Ishibashi et al, (1971) Virology 45:317; and Carter et al., (1983) Virology 126:505. For example, reports show that E1A and E4 regions are likely required for AAV replication, either directly or indirectly. Laughlin et al., (1982) J. Virol 41:868; Janik et al., (1981) Proc. Natl. Acad. Sci. USA 78:1925; Carter et al., (1983) Virology 126:505. In addition, International Publication WO 97/17458 and Matshushita et al., (1998) Gene Therapy 5:938 945, describe accessory function vectors encoding various adenoviral genes.

Infection of the host cell with a helper virus, or transfection of the host cell with an accessory function vector, allows expression of the accessory functions which transactivate the AAV helper construct to produce AAV Rep and/or Cap proteins. The Rep expression products in turn excise the recombinant DNA (including the DNA of interest, *e*.*g*., SERCA2) from the AAV expression vector. The Rep proteins also serve to duplicate the AAV genome. The expressed Cap proteins assemble into capsids, and the recombinant AAV genome is packaged into the capsids, AAV replication proceeds, and the DNA is packaged into rAAV virions.

Following recombinant AAV replication, rAAV virions can be purified from the host cell using a variety of conventional purification methods, such as column chromatography, CsCl gradients, and the like. For example, a plurality of column purification steps can be used, such as purification over an anion exchange column, an affinity column and/or a cation exchange column. See, for example, International Publication No. WO 02/12455. Further, if infection is employed to express the accessory functions, residual helper virus can be inactivated, using known methods. For example, adenovirus can be inactivated by heating to temperatures of approximately 60°C. for, *e.g.,* 20 minutes or more. This treatment effectively inactivates only the helper virus since AAV is extremely heat stable while the helper adenovirus is heat labile.

The resulting rAAV virions containing the SERCA2a nucleotide sequence of interest, or fragment, or functional fragment, or portion thereof can then be used for gene delivery using the techniques described below.

Recombinant AAV virions may be introduced into smooth muscle cells using either *in vivo* or *in vitro* (also termed *ex vivo*) transduction techniques. If transduced *in vitro,* the desired recipient cell, preferably a muscle cell, will be removed from the subject, transduced with rAAV virions and reintroduced into the subject. Alternatively, syngeneic or xenogeneic cells can be used where those cells will not generate an inappropriate immune response in the subject.

Suitable methods for the delivery and introduction of transduced cells into a subject have been described. For example, cells can be transduced *in vitro* by combining recombinant AAV virions (rAAV) with cells to be transduced in appropriate media, and those cells harboring the DNA of interest can be screened using conventional techniques such as Southern blots and/or PCR, or by using selectable markers. Transduced cells can then be formulated into pharmaceutical compositions, as described above, and the composition introduced into the subject by various techniques as described below, in one or more doses.

Recombinant AAV (rAAV) virions or cells transduced *in vitro* may be delivered directly to muscle by injection with a needle, catheter or related device, using techniques known in the art. For *in vivo* delivery, the rAAV virions will be formulated into pharmaceutical compositions and one or more dosages may be administered directly in the indicated manner. A therapeutically effective dose will include on the order of from about 10⁸/kg to 10¹⁶/kg of the rAAV virions, more preferably 10¹⁰/kg to 10¹⁴/kg, and even more preferably about 10¹¹/kg to 10¹³/kg of the rAAV virions (or viral genomes, also termed "vg" or "v.g."), or any value within these ranges.

One mode of administration of recombinant AAV virions uses a convection-enhanced delivery (CED) system. In this way, recombinant virions can be delivered to many cells over large areas of muscle. Moreover, the delivered vectors efficiently express transgenes in muscle cells. Any convection-enhanced delivery device may be appropriate for delivery of viral vectors. In a preferred embodiment, the device is an osmotic pump or an infusion pump. Both osmotic and infusion pumps are commercially available from a variety of suppliers, for example Alzet Corporation, Hamilton Corporation, Alza, Inc., Palo Alto, Calif.). Typically, a viral vector is delivered via CED devices as follows. A catheter, cannula or other injection device is inserted into appropriate muscle tissue in the chosen subject, such as skeletal muscle. For a detailed description regarding CED delivery, see U.S. Pat. No. 6,309,634.

Although, a perfusion method is described herein, various perfusion methods are available and standard in the art, and without being held to any one method, any perfusion method which gives the desired result is anticipated, such as a methods utilizing a catheter. The objective of the perfusion methods is to increase the time of contact between the vector *(e.g.,* adenovirus, AAV, lentivirus vectors) and the target cells (*e*.*g*., smooth muscle cells). Hence, the invention encompasses perfusion methods such as closed-circuit perfusion methods carried out at body temperature, and under defined conditions at, for example, 37°C, for about 2, 5, 10, 12, 15, 30, 60 or more minutes, or in larger animals or humans for about 2, 4, 6, 8, 10, 12 or more hours, allowing viral entry into the target cells and to create optimal conditions for gene expression and protein synthesis. For this reason, various excipients, e.g., natural and unnatural amino acids, growth factors and the like may be added to provide enough material for protein synthesis.

To determine efficacy in animal models which received the AAV-SERCA2 or AAV-SERCA2a compositions, the effect on pulmonary artery pressure and cardiac function may be determined using a pressure-volume measurement system post-injection e.g., one week or one month post-injection. Repeated measurements may be continued in order to monitor the short-term and long-terms effects and efficacy of the therapy. For example, if after one week the results are considered not significant, then new trials using higher (or lower) dosages, or multiple dosages may be performed.

Studies may be performed to determine gene expression including removal of pulmonary tissues receiving the perfusion as well as surrounding and/or control tissues. The tissues may be histologically processed by methods standard in the art, *e*.*g*., fixation methods, vibratome or cutting methods, and the like. For example, to study gene expression of SERCA2 or SERCA2a or other SERCA2 isoforms, immunohistochemistry using a SERCA2 or SERCA2a polynucleotides or antibodies which are immunogenic against SERCA2 or SERCA2 polypeptides may be performed. Further, for those animals receiving the AAV-GFP compositions, expression of GFP in the tissues may be measured using fluorescent microscopy or any other method standard in the art which can measure and detect fluorescence.

The present invention is more particularly described in the following examples which are intended as illustrative only since numerous modifications and variations therein will be apparent to those skilled in the art. The following examples are intended to illustrate but not limit the invention.

### EXAMPLE 1

Gene transfer (introduction of exogenous gene using viral or non-viral vector) using adeno-associated virus (AAV: type of virus) is well known for it safety and ability to express exogenous genes for a long period. SERCA2a overexpression using adeno virus wass shown to prevent remodeling of smooth muscle cell in coronary artery and increase relaxation and production of eNOS, which is well-known vasodilator. (Hadri L, Circ Res 2007;101 :E65-E65). As such, it is hypothesized that the overexpression of SERCA2a on the pulmonary artery using an adeno-associated virus prevents the remodeling of smooth muscle cell on the pulmonary artery and the production of eNOS to decrease vasoconstriction.

The experiment is performed on control and pulmonary hypertension, as well as pulmonary hypertension with gene therapy or control virus injection, measures cardiac contractility and hemodynamic including pulmonary arterial resistance and sytemic arterial resistance. Monocrotaline (MCT) induced PAH is a clinically relevant and easy to perform PAH model in rats.

Pulmonary hypertension was induced in the male Sprague-Dawley rats (weight range from 250-300 gm) by subcutaneous injection of monocrotaline (MCT), which will be dissolved in 0.1 N HCl and neutralized with 1N NaOH (pH = 7.4) and administered at a dose of 50mg/kg. At the same time, AAV1.SERCA2a was delivered via a catheter within the tracheal tube into the lung while the animal was breathing spontaneously. The effect on pulmonary artery pressure and cardiac function was determined one month later with a pressure-volume measurement system. HN buffer will be used for MCT control, and saline will be used for AAV control.

The control (n=8) and the treatment groups (n=8) were comparable in their bodyweight at baseline. Efficient transfection was confirmed with immuno-histochemistry. Catheterization of the pulmonary artery and right ventricle showed significantly lower systolic pressures in treated animals (36.6 ± 3.6 vs. 45.9 ± 4.5 mmHg, P < 0.05) (see Figures 1A-1D) as well as right ventricular diastolic pressures (3.0 ± 1.1 vs. 4.6 ± 1.3 mmHg, P < 0.05). Left ventricular pressures remained unchanged (systolic: 94.7 ± 6.0 vs. 101.4 ± 5.5 mmHg) as well as dP/dtMax (5775 ± 589.2 vs. 6404 ± 818.7 mmHg/s). Right ventricular weight at sacrifice at one month was significantly reduced in the SERCA2a group (0.32 ± 0.02 vs. 0.39 ± 0.03 g, P < 0.05). The weight of the left ventricle adjusted to bodyweight did not differ among groups.

### EXAMPLE 2

Studies with animals were approved by the Institutional Animal Care and Use Committee (IACUC). All of the animal-related procedures were performed according to the Guidelines of IACUC. Male Sprague-Dawley rats (weight range from 250-300 gm) or Wistar rats (weight range from 90-150 gm) will be divided to Injection or Perfusion groups by the transfection method and subdivided into 4 subgroups: Control + Saline (Control group, n=4), Control + AAV1. GFP (GFP group, n=4), MCT-induced PH + AAV1.SERCA2a (SERCA2a group, n=6), and MCT-induced PH + Saline (Saline group, n=6).

An additional study will be performed in which the animals are divided as follows:
(A) MCT PAH group with Intra-tracheal injection
   AAV.SERCA2a group (n=40)
   AAV.eNOS group (n=40)
   AAV. GFP or beta-gal group (n=40)
(B) MCT PAH group with jugular vein injection
   AAV.SERCA2a group (n=40)
   AAV.eNOS group (n=40)
   AAV.GFP or beta-gal group (n=40)

*Intra-Tracheal Administration of AAV -* The procedure is performed on male Wistar rats (BW = 100g) or Spraque-Dawley rats (BW = 250-300g). Rats receive general anesthesia (isoflurane 0.5-1.5% in 100% oxygen via mask). After endotracheal intubation, mechanical ventilation is initiated. After general condition is stabilized, a small silicone elastomer catheter will be advanced through the intubation tube and be used to administer AAV into the lungs.

*Jugular Vein Administration of AAV* - As above, the procedure is performed on 100 gram male Wistar rats. However, instead of using intra-tracheal administration, AAV will be administered by jugular vein injection. The neck will be prepared with 70% isopropyl alcohol followed by providone iodine. Cut-down will be performed to expose jugular vein and jugular vein will be punctured with 26G needle. AAV (400µl) will be slowly injected into the jugular vein.

*Induction of PAH -* Animals will receive MCT (40 mg/kg) via subcutaneous injection at Day 28.

*Echocardiography -* Animals will receive sedation with ketamine 80-100mg/kg IP since no pain is involved in this procedure, only sedation is needed to prevent motion artifact and other anesthetics can interfere significantly with heart rate or cardiac contractility and loading. Cardiac function will be assessed by echocardiography. From the left anterior chest wall, an echo probe will be applied. Dilatation of cardiac chambers and decreased cardiac contractility will be assessed.

Between weeks 4 and 12 the animals will be monitored twice a week and they will be evaluated for signs of severe heart failure and dyspnea such as 1) impaired activity, 2) reluctance to move, 3) shaking, 4) decrease oral intake, 5) frothing at the mouth, 6) audible wheezing, 7) massive ascites. If they display any of the clinical signs mentioned above they will be sacrificed. Maximum expression of an exogenous protein is achieved 6 to 8 weeks after AAV administration. MCT-PAH is fully developed at 3 to 4 weeks after MCT injection. As such, at least for 28 days are necessary to obtain maximum expression of exogenous protein by the time MCT injected rat starts to show symptoms. Also, we have to wait 28 to 56 days after MCT injection to obtain clinically significant PAH.

*Hemodynamic measurement using Scisense pressure-volume catheters -* Anesthesia is induced in an isoflurane chamber and, following induction, animals are ventilated with a mixture of oxygen and isoflurane 2-5% through a nose cone. After deep stable anesthesia is obtained, animals are intubated and isoflurane 2-5% is administered through mechanical ventilation, together with oxygen. The chest will be opened. A Scisense pressure-volume catheter or Millar catheter will be introduced into the left and right ventricule. The measurement of mean right atrium, pulmonary artery, left atrium, and aorta will be measured with this catheter. Cardiac output will be measured at the same time using this catheter. During hemodynamic measurements, isoflurane will be titrated both to maintain general anesthesia and to achieve a heart rate as close as possible to physiologic heart rates. Often, this is possible with only 1% of isoflurane. At the end of the procedure, the left or right jugular vein are canulated and 50 microliters of 30% sodium chloride solution is injected to calibrate the volume sensors. The animals are sacrificed at the end of the procedure; the heart and the lungs being surgically removed under deep general anesthesia with isoflurane 5%. PVR (dynes· sec· cm-5) = (mPAP-mLA)/ CO*80; SVR (dynes· sec· cm-5) = (mSAP-mRA)/CO*80.

Body weight monitoring after gene therapy (if applicable) and MCT administration occurs throughout the experiment. Body weight is measured and recorded before every surgery, gene therapy and before every echocardiography to monitor animal growth and wellbeing and to index cardiac size.

Although the present invention has been described with reference to specific details of certain embodiments thereof in the above examples, it will be understood that modifications and variations are encompassed within the scope of the invention. Accordingly, the invention is limited only by the following claims.

## Claims

1. An adeno-associated viral (AAV) expression vector for use in treating pulmonary hypertension, comprising a sarcoplasmic reticulum (SR) calcium ⁺⁺ ATPase (SERCA) transgene, wherein the transgene is SERCA isoform 2a (SERCA2a) and modulates Ca²⁺ ion transport, and wherein expression of the transgene increases host cell function in a subject.

2. The vector for use as claimed in claim 1, wherein the host cells are associated with pulmonary function.

3. The vector for use as claimed in claim 2, wherein the host cells are selected from the group consisting of pulmonary artery muscle cells, pulmonary vein muscle cells, pulmonary capillary muscle cells, pulmonary arterioles, alveolae, and vascular smooth muscle cells.

4. The vector for use as claimed in claim 1, 2 or 3, wherein the vector is serotype 1 (AAV1), serotype 2 (AAV2), or serotype 8 (AAV8).

5. The vector for use as claimed in claim 1, wherein the treatment decreases blood pressure in the pulmonary artery or the pulmonary vein, results in lower systolic pressure and ventricular diastolic pressure, improves pulmonary vascular remodeling, decreases proliferation of vascular smooth muscle cells, or increases production of eNOS from endothelial cells.

6. The vector for use as claimed in any one of claims 1-4, wherein the subject is a mammal.

7. The vector for use as claimed in claim 6, wherein the mammal is a human.

8. The vector for use as claimed in any one of claims 1-4 and 6, wherein the vector is delivered by a method selected from the group consisting of intranasal spray, inhalation, and aerosol delivery.

9. The vector for use as claimed in any one of claims 1-4, 6 and 7, wherein the vector is delivered in the form of a microparticle.

10. A recombinant adeno-associated virus (AAV) virion for use in treating pulmonary hypertension, wherein (a) the virion comprises an AAV vector comprising a transgene operably linked to control elements that direct expression of the transgene in a host cell, (b) the expression of the transgene improves lung function, and (c) the transgene is sarcoplasmic reticulum (SR) calcium ⁺⁺ ATPase (SERCA) isoform 2a (SERCA2a).

11. A recombinant AAV virion (rAAV) for use in treating pulmonary hypertension, the rAAV comprising a polynucleotide encoding a protein, wherein (a) the polynucleotide is operably linked to a control element capable of directing expression of the protein, (b) the rAAV virion is delivered directly into a pulmonary muscle cell of a mammalian subject, (c) the protein is sarcoplasmic reticulum (SR) calcium ⁺⁺ ATPase isoform 2a (SERCA2a) and (d) the protein is expressed at a therapeutically effective level in the muscle cell.

12. The recombinant AAV virion for use as claimed claim 11, wherein the subject is a human.

13. The recombinant AAV virion for use as claimed in any one of claims 10-12, wherein the virion is delivered by a method selected from the group consisting of intranasal spray, inhalation, and aerosol delivery.

14. The recombinant AAV virion for use as claimed in any one of claims10-13, wherein the virion is delivered in the form of a microparticle.

## Patentansprüche

1. AAV (adeno-assoziiertes Virus)-Expressionsvektor zur Verwendung bei der Behandlung von pulmonaler Hypertonie, umfassend ein SERCA-Transgen (SERCA = Calcium⁺⁺-ATPase des sarkoplasmatischen Retikulums), bei dem es sich um die SERCA-Isoform 2a (SERCA2a) handelt und das den Ca²⁺-Ionentransport moduliert, und wobei die Expression des Transgens die Funktion der Wirtszellen in einem Subjekt steigert.

2. Vektor zur Verwendung nach Anspruch 1, wobei die Wirtszellen mit der pulmonalen Funktion assoziiert sind.

3. Vektor zur Verwendung nach Anspruch 2, wobei die Wirtszellen ausgewählt sind aus der Gruppe bestehend aus pulmonalen arteriellen Muskelzellen, pulmonalen venösen Muskelzellen, pulmonalen kapillaren Muskelzellen, pulmonalen Arteriolen, Alveolen und Zellen der glatten Gefäßmuskulatur.

4. Vektor zur Verwendung nach Anspruch 1, 2 oder 3, wobei es sich bei dem Vektor um Serotyp 1 (AAV1), Serotyp 2 (AAV2) oder Serotyp 8 (AAV8) handelt.

5. Vektor zur Verwendung nach Anspruch 1, wobei die Behandlung den Blutdruck in der pulmonalen Arterie oder der pulmonalen Vene senkt, zu einer Absenkung des systolischen Drucks und des ventrikulären diastolischen Drucks führt, die Remodellierung der pulmonalen Vaskulatur verbessert, die Proliferation von Zellen der glatten Gefäßmuskulatur verringert oder die Produktion von eNOS in den Endothelzellen steigert.

6. Vektor zur Verwendung nach einem der Ansprüche 1 bis 4, wobei es sich bei dem Subjekt um einen Säuger handelt.

7. Vektor zur Verwendung nach Anspruch 6, wobei es sich bei dem Säuger um einen Menschen handelt.

8. Vektor zur Verwendung nach einem der Ansprüche 1 bis 4 und 6, wobei die Zufuhr des Vektors gemäß einem Verfahren erfolgt, das ausgewählt ist aus der Gruppe bestehend aus Einsprühen in die Nase, Inhalation und aerosolischer Verabreichung.

9. Vektor zur Verwendung nach einem der Ansprüche 1 bis 4, 6 und 7, wobei der Vektor in Form eines Mikropartikels zugeführt wird.

10. Rekombinantes AAV (adeno-assoziiertes Virus)-Virion zur Verwendung bei der Behandlung von pulmonaler Hypertonie, wobei (a) das Virion einen AAV-Vektor umfasst, der ein Transgen umfasst, das funktionell mit Kontrollelementen verknüpft ist, welche die Expression des Transgens in einer Wirtszelle regulieren, (b) die Expression des Transgens die Lungenfunktion verbessert und (c) es sich bei dem Transgen um Calcium⁺⁺-ATPase des sarkoplasmatischen Retikulums (SR) (= SERCA) der Isoform 2a (SERCA2a) handelt.

11. Rekombinantes AAV-Virion (rAAV) zur Verwendung bei der Behandlung von pulmonaler Hypertonie, wobei das rAAV ein für ein Protein kodierendes Polynukleotid umfasst, wobei (a) das Polynukleotid funktionell mit einem Kontrollelement verknüpft ist, das zur Regulierung der Expression des Proteins in der Lage ist, (b) das rAAV-Virion direkt in eine pulmonale Muskelzelle eines Säugersubjekts abgegeben wird, (c) es sich bei dem Protein um Calcium⁺⁺-ATPase des sarkoplasmatischen Retikulums (SR) (= SERCA) der Isoform 2a (SERCA2a) handelt und (d) die Expression des Proteins in der Muskelzelle auf einem therapeutisch wirksamen Niveau erfolgt.

12. Rekombinantes AAV-Virion zur Verwendung nach Anspruch 11, wobei es sich bei dem Subjekt um einen Menschen handelt.

13. Rekombinantes AAV-Virion zur Verwendung nach einem der Ansprüche 10 bis 12, wobei die Zufuhr des Virions gemäß einem Verfahren erfolgt, das ausgewählt ist aus der Gruppe bestehend aus Einsprühen in die Nase, Inhalation und aerosolischer Verabreichung.

14. Rekombinantes AAV-Virion zur Verwendung nach einem der Ansprüche 10 bis 13, wobei das Virion in Form eines Mikropartikels zugeführt wird.

## Revendications

1. Vecteur d'expression viral adéno-associé (AAV) pour l'utilisation dans le traitement de l'hypertension pulmonaire, comprenant un transgène calcium⁺⁺ ATPase du réticulum sarcoplasmique (SR) (SERCA), dans lequel le transgène est l'isoforme 2a de SERCA (SERCA2a) et module le transport d'ions Ca²⁺, et dans lequel l'expression du transgène augmente la fonction de cellule hôte chez un sujet.

2. Vecteur pour l'utilisation telle que revendiquée dans la revendication 1, dans lequel les cellules hôtes sont associées à la fonction pulmonaire.

3. Vecteur pour l'utilisation telle que revendiquée dans la revendication 2, dans lequel les cellules hôtes sont choisies dans le groupe consistant en cellules musculaires de l'artère pulmonaire, cellules musculaires de la veine pulmonaire, cellules musculaires de capillaire pulmonaire, artérioles pulmonaires, alvéoles et cellules musculaires lisses vasculaires.

4. Vecteur pour l'utilisation telle que revendiquée dans l'une des revendications 1, 2 ou 3, dans lequel le vecteur est de sérotype 1 (AAV1), sérotype 2 (AAV2) ou sérotype 8 (AAV8).

5. Vecteur pour l'utilisation telle que revendiquée dans la revendication 1, dans lequel le traitement diminue la pression sanguine dans l'artère pulmonaire ou la veine pulmonaire, conduit à une pression systolique inférieure et à une pression diastolique ventriculaire inférieure, améliore le remodelage vasculaire pulmonaire, diminue la prolifération des cellules musculaires lisses vasculaires ou augmente la production de eNOS à partir des cellules endothéliales.

6. Vecteur pour l'utilisation telle que revendiquée dans l'une quelconque des revendications 1-4, dans lequel le sujet est un mammifère.

7. Vecteur pour l'utilisation telle que revendiquée dans la revendication 6, dans lequel le mammifère est un être humain.

8. Vecteur pour l'utilisation telle que revendiquée dans l'une quelconque des revendications 1-4 et 6, dans lequel le vecteur est administré par un procédé choisi dans le groupe consistant en vaporisation instranasale, inhalation et administration par aérosol.

9. Vecteur pour l'utilisation telle que revendiquée dans l'une quelconque des revendications 1-4, 6 et 7, dans lequel le vecteur est administré sous la forme d'une microparticule.

10. Virion de virus adéno-associé (AAV) recombinant pour l'utilisation dans le traitement de l'hypertension pulmonaire, dans lequel (a) le virion comprend un vecteur AAV comprenant un transgène lié fonctionnellement à des éléments de contrôle qui dirigent l'expression du transgène dans une cellule hôte, (b) l'expression du transgène améliore la fonction pulmonaire et (c) le transgène est l'isoforme 2a de calcium⁺⁺ ATPase du réticulum sarcoplasmique (SR) (SERCA) (SERCA2a).

11. Virion AAV recombinant (rAAV) pour l'utilisation dans le traitement de l'hypertension pulmonaire, le rAAV comprenant un polynucléotide codant pour une protéine, dans lequel (a) le polynucléotide est lié fonctionnellement à un élément de contrôle capable de diriger l'expression de la protéine, (b) le virion rAAV est administré directement dans une cellule musculaire pulmonaire d'un sujet mammifère, (c) la protéine est l'isoforme 2a de calcium⁺⁺ ATPase du réticulum sarcoplasmique (SR) (SERCA2a) et (d) la protéine est exprimée à un taux thérapeutiquement efficace dans la cellule musculaire.

12. Virion AAV recombinant pour l'utilisation telle que revendiquée dans la revendication 11, dans lequel le sujet est un être humain.

13. Virion AAV recombinant pour l'utilisation telle que revendiquée dans l'une quelconque des revendications 10-12, dans lequel le virion est administré par un procédé choisi dans le groupe consistant en une vaporisation intranasale, une inhalation et une administration par aérosol.

14. Virion AAV recombinant pour l'utilisation telle que revendiquée dans l'une quelconque des revendications 10-13, dans lequel le virion est administré sous la forme d'une microparticule.
